# EUROPÄISCHE PATENTSCHRIFT

(11) **EP 4 216 944 B1**
(45) Veröffentlichungstag und Bekanntmachung des Hinweises auf die Patenterteilung: **07.08.2024**
(21) Anmeldenummer: 21786963.5
(22) Anmeldetag: 06.10.2021
(51) Int. Cl.: A61K 31/352, A61K 31/573, A61K 45/06, A61P 31/14

(54) **NEUES THERAPIEKONZEPT FÜR DIE BEHANDLUNG VON CORONA-INFEKTIONEN, INSBESONDERE COVID-19-INFEKTIONEN**
NEW THERAPY CONCEPT FOR THE TREATMENT OF CORONA INFECTIONS, MORE PARTICULARLY COVID-19 INFECTIONS
NOUVEAU CONCEPT THÉRAPEUTIQUE POUR LE TRAITEMENT D'INFECTIONS À CORONAVIRUS, PLUS PARTICULIÈREMENT D'INFECTIONS COVID-19

(30) Priorität: 18.11.2020 DE 102020007038; 30.11.2020 DE 102020131716
(43) Veröffentlichungstag der Anmeldung: 02.08.2023
(73) Patentinhaber: Maria Clementine Martin Klosterfrau Vertriebsgesellschaft mbH, 50670 Köln (DE)
(72) Erfinder: PLOCH, Michael, 50670 Köln (DE)
(74) Vertreter: Von Rohr Patentanwälte Partnerschaft mbB
(86) Internationale Anmeldenummer: PCT/EP2021/077544
(87) Internationale Veröffentlichungsnummer: WO 2022/106112

(56) Entgegenhaltungen:
- WO-A1-2010/025821
- WO-A1-2021/216749
- SHARMA ARUN DEV ET AL: "Eucalyptol (1,8 cineole) from Eucalyptus Essential Oil a Potential Inhibitor of COVID 19 Corona Virus Infection by Molecular Docking Studies<strong> </strong>", PRERPINTS, 30 March 2020 (2020-03-30), pages 1 - 8, XP055865125, Retrieved from the Internet <URL:https://www.preprints.org/manuscript/202003.0455/v1> [retrieved on 20211124], DOI: 10.20944/preprints202003.0455.v1
- MY TRAN THI AI ET AL, CHEMISTRYSELECT, vol. 5, no. 21, 8 June 2020 (2020-06-08), DE, pages 6312 - 6320, XP055815069, ISSN: 2365-6549, Retrieved from the Internet <URL:https://onlinelibrary.wiley.com/doi/full-xml/10.1002/slct.202000822> DOI: 10.1002/slct.202000822
- ASIF MUHAMMAD ET AL: "COVID-19 and therapy with essential oils having antiviral, anti-inflammatory, and immunomodulatory properties", INFLAMMOPHARMACOLOGY, KLUWER ACADEMIC PUBLISHERS, DORDRECHT, NL, vol. 28, no. 5, 14 August 2020 (2020-08-14), pages 1153 - 1161, XP037257443, ISSN: 0925-4692, [retrieved on 20200814], DOI: 10.1007/S10787-020-00744-0
- LOIZZO M R ET AL: "Phytochemical Analysis and in vitro Antiviral Activities of the Essential Oils of Seven Lebanon Species", CHEMISTRY & BIODIVERSITY, HELVETICA CHIMICA ACTA, ZUERICH, CH, vol. 5, no. 3, 1 March 2008 (2008-03-01), pages 461 - 470, XP002558017, ISSN: 1612-1872, [retrieved on 20080320], DOI: 10.1002/CBDV.200890045

## Beschreibung

Die vorliegende Erfindung betrifft das technische (d. h. medizinisch-pharmazeutische) Gebiet der Therapie von viralen Infektionen (Virusinfektionen), insbesondere Infektionen mit Corona-Viren, insbesondere COVID-19. Insbesondere betrifft die vorliegende Erfindung einen Wirkstoff und ein Arzneimittel zur Verwendung für eine solche Therapie.

Insbesondere betrifft die vorliegende Erfindung einen Wirkstoff und ein Arzneimittel bzw. eine Zusammensetzung jeweils zur Verwendung bei der prophylaktischen und/oder therapeutischen Behandlung von durch Corona-Viren hervorgerufenen Virusinfektionen (nachfolgend auch als "Corona-Infektionen", "Corona(virus)infektionen" oder dergleichen bezeichnet)nämlich COVID-19 bzw. die Verwendung eines Wirkstoffs und eines Arzneimittels oder einer Zusammensetzung zur prophylaktischen und/oder therapeutischen Behandlung von durch Corona-Viren hervorgerufenen Virusinfektionen nämlich COVID-19. Im Rahmen der vorliegenden Erfindung kommt dabei als Wirkstoff 1 ,8-Cineol bzw. ein(e) diesen Wirkstoff enthaltendes Arzneimittel oder Zusammensetzung zum Einsatz, wie in der nachfolgenden Beschreibung und in den Patentansprüchen zu der vorliegenden Erfindung weiterführend definiert und beschrieben.

Coronaviridae ist eine Virusfamilie innerhalb der Ordnung Nidovirales. Die Viren innerhalb dieser Virusfamilie werden fachumgangssprachlich auch Coronaviren genannt und gehören zu den RNA-Viren mit den größten Genomen. Die ersten Coronaviren wurden bereits Mitte der 1960er Jahre entdeckt und beschrieben. Die im elektronenmikroskopischen Bild grob kugelförmigen Viren fallen durch einen Kranz blütenblattartiger Fortsätze auf, die an eine Sonnenkorona erinnern, welche dieser Virusfamilie ihren Namen gegeben hat.

Vertreter der Virenfamilie der Coronaviridae verursachen bei allen vier Klassen der Landwirbeltiere (d. h. Säugetiere, Vögel, Reptilien und Amphibien) sehr unterschiedliche Erkrankungen. Sie sind genetisch hochvariabel und können so auch mehrere Arten von Wirten infizieren.

Beim Menschen sind sieben Arten von Coronaviren als Erreger von leichten respiratorischen Infektionen (insbesondere Erkältungen bzw. grippalen Infekten) bis hin zum sogenannten schweren akuten Atemwegssyndrom (SARS bzw. *Severe Acute Respiratory Syndrome*) von Bedeutung.

Unter den menschlichen Coronaviren besonders bekannt geworden sind die folgenden Coronaviren: SARS-CoV-1 (*Severe Acute Respiratory Syndrome Coronavirus-*1), MERS-CoV (*Middle East Respiratory Syndrome Coronavirus*) und SARS-CoV-2 *(Severe Acute Respiratory Syndrome Coronavirus 2* bzw. COVID-19). Diese Coronaviren sind die Auslöser der SARS-Pandemie 2002/2003, der MERS-Epidemie 2012 bzw. der COVID-19-Pandemie ab 2019.

COVID-19 (d.h. die Abkürzung für Englisch *Coronavirus-Disease 2019* oder Deutsch Coronavirus-Krankheit-2019, umgangssprachlich auch als Coronavirus-Erkrankung/-Infektion oder dergleichen bezeichnet) ist eine Infektionskrankheit, in deren Folge es zu einer Infektion mit dem neuartigen Coronavirus SARS-CoV-2 kommt. Die primär die Atemwege befallende Erkrankung wurde erstmals Ende des Jahres 2019 in Wuhan/China beschrieben, entwickelte sich dann im Januar 2020 zunächst in der Volksrepublik China zu einer Epidemie und breitete sich schließlich weltweit zur COVID-19-Pandemie aus.

Die Ansteckung bei COVID-19 erfolgt in der Regel durch Tröpfchenübertragung. Die Inkubationszeit von COVID-19 beträgt durchschnittlich fünf bis sechs Tage, wobei zwischen Ansteckung und dem Auftreten erster Symptome bisweilen aber auch bis zu zwei Wochen vergehen können und vereinzelt erste Symptome schon innerhalb von 24 Stunden nach Ansteckung mit SARS-CoV-2 auftreten können. Die häufigsten Symptome sind Fieber, trockener Husten und Müdigkeit, weniger häufig sind dagegen Muskelschmerzen, eine verstopfte Nase, Kopfschmerzen, Bindehautentzündung, Halsschmerzen, Durchfall, Geschmacks- oder Geruchsverlust oder Hautausschlag oder Verfärbung von Fingern oder Zehen zu beobachten. Infizierte ohne Symptome können trotzdem potentielle Überträger des Coronavirus sein. Bei einem leichten Krankheitsverlauf klingen die Symptome im Allgemeinen innerhalb von zwei Wochen ab. Verläuft COVID-19 schwerer, kann die Rekonvaleszenz drei bis sechs Wochen oder sogar noch länger andauern.

Bei rund 81 % der registrierten Infektionen ist ein leichter Krankheitsverlauf mit Fieber oder einer leichten Lungenentzündung zu beobachten; bei etwa 14 % der Fälle ist jedoch der Verlauf schwerer, und in etwa 5 % der Fälle ist der Verlauf sogar derart schwer, dass eine intensivstationäre Betreuung der Patienten erfolgen muss.

COVID-19 ist zurzeit Gegenstand intensiver Forschung. Effiziente und spezifische kausale Therapien oder Impfungen stehen zurzeit noch nicht bzw. nur eingeschränkt zur Verfügung.

Das COVID-19 auslösende Virus SARS-CoV-2 dringt - wie auch SARS-CoV-1 bei SARS - typischerweise über eine Bindung an das in der Zellmembran verankerte Enzym ACE2 (Angiotensin-konvertierendes Enzym 2) in die menschliche Zelle ein, wobei das virale Spike-Protein mit ACE2 interagiert. Für diesen Prozess ist die Mitwirkung der Serinprotease TMPRSS2 (Transmembrane Serinprotease 2) erforderlich. Im Versuch mit HeLa-Zellen, welche ACE2 des Menschen, der Chinesischen Hufeisennase (Rhinolophus sinicus), einer Schleichkatzenart, des Hausschweins und der Maus exprimieren, konnte SARS-CoV-2 das jeweilige ACE2-Protein als Rezeptor nutzen, um in die Zelle einzudringen; nur bei dem Maus-ACE2-Enzym gelang dies nicht - ebenso wenig bei HeLa-Zellen, welche kein ACE2 bilden. An Rezeptoren, welche von anderen Coronaviren genutzt werden, findet dagegen keine Bindung von SARS-CoV-2 statt.

Neben dem Weg über ACE2 wurden bei T-Lymphozyten, welche kein oder wenig ACE2 auf ihrer Oberfläche tragen, ein alternativer Eindringweg experimentell nachgewiesen: Das Virus dringt bei diesen Zellen über eine durch das Spike-Protein vermittelte Verschmelzung der Virusmembran mit der Lymphozytenzellmembran ein.

Eine reverse Suche in einer humanen Gendatenbank (*Human Cell Atlas* oder kurz *HCA*) nach Zelltyp und Geweben, bei welchen neben ACE2 auch TMPRSS₂ auf Membranoberflächen vorhanden ist, zeigt, dass in der Nasenschleimhaut, insbesondere den Becherzellen, aber auch den Flimmerepithelen, die höchsten Konzentrationen dieser beiden Proteine auftreten, weshalb diese Zellen als Eintrittspforte für SARS-CoV-2 angesehen und auch als Reservoir vermutet werden. Die Proteine werden ebenso in den Hornhaut-Zellen des Auges, in der Darmschleimhaut sowie im Herzen in Perizyten der Blutkapillaren, Herzmuskelzellen und Fibroblasten gebildet. Dabei bleibt die erste Phase des Befalls im Nasenrachen nahezu symptomfrei, wohingegen bei Übergang in eine schwere Verlaufsform überwiegend die Lunge angegriffen wird, da ein Großteil der ACE-2-exprimierenden Zellen des Menschen in den Typ-II-Pneumozyten der Lunge vorkommt. Als weitere Gründe für die besondere Anfälligkeit der Lunge wird ihre große Oberfläche angegeben; außerdem verfügen die ACE-2-exprimierenden Pneumozyt-Typ-II-Zellen über diverse Gene, welche die Replikation und Transmission von SARS-CoV-2 begünstigen.

Bei Untersuchungen an kryokonservierten Lungengewebsproben von Nichtinfizierten kann auch gezeigt werden, dass Lungengewebe kaum ACE2 sowie die Transmembranprotease TMPRSS2 ausbildet, die Pneumozyten Typ II in der Lunge hingegen vermehrt. Diese Vorläuferzellen sind bei Männern und in fortgeschrittenem Alter tendenziell vermehrt nachzuweisen. Neben unterschiedlichen ACE2-Werten bei Männern und Frauen wird eine Ursache für die unterschiedliche schwere der Erkrankung im geschlechtsspezifischen Hormonhaushalt vermutet: Östrogen fördert eine Immunantwort, wohingegen Testosteron diese unterdrückt.

Neuere Erkenntnisse zeigen auch, dass im Lungenepithel und benachbarten Gewebezellen die Proprotease Furin coexprimiert wird, was wiederum dem Virus den Zellzutritt vereinfacht, da es am Spike-Protein eine Furin-spezifische Trennstelle aufweist.

Außer in der Lunge wurde ACE2 auch im Dünn- und Dickdarm, in den Atemwegen und in den Nieren nachgewiesen. Eine Vermehrung des Virus in Darmzellen wurde ebenfalls bestätigt.

Was klinische Symptome und laborchemische Krankheitszeichen anbelangt, so ist eine Abgrenzung von anderen Viruserkrankungen, wie etwa Influenza, allein anhand der Symptome schwierig. Nach einer Inkubationszeit von typischerweise fünf bis sechs Tagen, in seltenen Fällen bis zu 14 Tagen, können - wie eingangs beschrieben - Fieber, Muskelschmerzen und trockener Husten auftreten. Häufig manifestiert sich die Krankheit auch mit allgemeinem schwerem Krankheitsgefühl. Im weiteren Verlauf kann sich dann eine schwere Atemnot aufgrund einer Infektion der unteren Atemwege bis zur Lungenentzündung entwickeln. Diese kann mit Brustschmerzen im Sinne einer Pleuritis einhergehen. Etwa 85 % der schwer erkrankten COVID-19-Patienten entwickeln eine Lymphopenie. Die schwer erkrankten Patienten entwickeln häufig zudem eine Hyperzytokinämie (sogenannter Zytokinsturm), wobei dieser Zytokinsturm durch eine Überreaktion des Immunsystems entsteht; diese Überreaktion ist durch einen deutlichen Anstieg von entzündungsrelevanten Zytokinen gekennzeichnet, wie insbesondere Interleukin-6, Interleukin-8, Interleukin-1beta und TNF-alpha. Die verstärkte Freisetzung dieser Zytokine führt zu einer Überproduktion von Immunzellen, vor allem im Lungengewebe.

Die Diagnose von COVID-19 kann insbesondere durch labordiagnostischen Nachweis erfolgen, insbesondere mittels spezifischer Virus- und Antikörpernachweise.

Eine effiziente und spezifische kausale Therapie konnte bislang noch nicht etabliert werden. Das Nukleosidanalogon Remdesivir zeigt in einer vorläufig veröffentlichten randomisierten Studie eine Verkürzung der Krankheitsdauer bei hospitalisierten Patienten. Dieses Medikament ist in der Europäischen Union für COVID-19-Patienten, welche Sauerstoff benötigen, zugelassen und kann laut Leitlinie der DIVI (Deutsche Interdisziplinäre Vereinigung für Intensiv- und Notfallmedizin e.V.) bei schwerkranken Patienten erwogen werden.

Weitere Studien belegen, dass Dexamethason (9-Fluor-16alpha-methylprednisolon) die Sterberate bei Patienten an Beatmungsgeräten von 41 % auf 29 % und bei Patienten mit Sauerstoffversorgung von 26 % auf 23 % reduziert. Dexamethason vermag eine überschießende Reaktion des Immunsystems, insbesondere den sogenannten Zytokinsturm, zu verhindern bzw. zu vermindern.

Bei schwer erkrankten COVID-19-Patienten wird zudem eine Gabe von niedrigmolekularem Heparin empfohlen, um das Risiko von Thrombosen und Lungenembolien zu senken. Aufgrund des hohen Vorkommens von Lungenembolien und Beinvenenthrombosen in Verbindung mit schweren Verläufen von COVID-19 wird die Gabe stärker gerinnungshemmender Präparate in Erwägung gezogen; diesbezüglich liegen jedoch noch keine ausreichenden Daten über Nutzen und Risiko vor.

Chloroquin und Hydroxychloroquin dagegen zeigen - entgegen ursprünglicher Erwartungen - keine Hinweise auf eine Wirksamkeit.

Tocilizumab, ein monoklonaler Antikörper, welcher unter anderem zur Behandlung verschiedener Formen von rheumatoider Arthritis und dem Zytokin-Freisetzungssyndrom zugelassen ist, hat sich ebenfalls als unwirksam erwiesen.

Antikörperreiches Plasma genesener Patienten dagegen scheint geeignet zu sein, um Akutfälle zu therapieren, kann aber einen Erfolg nur in der Frühphase der Erkrankung belegen.

Bisher ist auch noch unklar, inwieweit eine durchgemachte Infektion eine Immunität verleiht; bislang wird auf Basis von Erkenntnissen aus Tiermodellen davon ausgegangen, dass eine akute Immunität besteht, wobei jedoch nicht feststeht, wie lange diese andauert. Auch der Forschungsstand zu Spätfolgen und zur Abschätzung des Sterberisikos ist noch ungewiss.

Im Ergebnis zielen daher die weltweit meisten Maßnahmen auf einer Prävention von COVID-19-Erkrankungen ab, insbesondere durch verstärkte individuelle Hygienemaßnahmen, wie das Tragen eines Mund-Nasen-Schutzes, Handdesinfektion, Flächenreinigung etc., da bislang eine etablierte kausale Therapie zur kurativen Behandlung oder eine Impfung zu Zwecken der Prävention noch nicht existiert.

Die wissenschaftliche Veröffentlichung Sharma Arun Dev et al.: "Eucalyptol (1,8 cineole) from Eucalyptus Essential Oil a Potential Inhibitor of COVID 19 Corona Virus Infection by Molecular Docking Studies ", abgerufen auf www.preprints.org, erschienen am 30. März 2010, untersucht in Form von computerbasierten Simulationen bzw. in-silico-Untersuchungen einen möglichen Effekt von Eucalyptol auf bestimmte Proteine, speziell auf eine Protease (Mpro) von COVID-19.

Darüber hinaus untersucht die wissenschaftliche Veröffentlichung My Tran Thi Ai et al.: "Evaluation of the Inhibitory Activities of COVID-19 of Melaleuca cajuputi Oil Using Docking Simulation", ChemistrySelect, Bd. 5, Nr. 21, 8. Juni 2020, Seiten 6312 bis 6320) Untersuchungen von möglichen inhibitorischen Eigenschaften eines ätherischen Öls von *Melaleuca cajuputi* in Bezug auf COVID-19 auf Basis einer computerbasierten Simulation ("*docking simulation*")*.* Es wird eine Vielzahl an Bestandteilen des ätherischen Öls in der Computersimulation gegenüber dem sogenannten humanen ACE2-Protein und der Protease PDB6LU7 von SARS-CoV-2 als jeweilige Bindungskomponenten untersucht.

Die wissenschaftliche Veröffentlichung Asif Muhammad et al.: "COVID-19 and therapy with essential oils having antiviral, anti-inflammatory, and immunomodulatory properties", Inflammopharmacology, Bd. 28, Nr. 5, August 2020, Seiten 1153 bis 1161) betrifft zudem einen Review-Artikel zu COVID-19 zu einer möglichen Therapie mit ätherischen Ölen, welche antivirale, antiinflammatorische und immunmodulatorische Eigenschaften aufweisen.

Weiterhin beschreibt die wissenschaftliche Veröffentlichung Loizzo M R et al.: "Phytochemical Analysis and in vitro Antiviral Activities of the Essential Oils of Seven Lebanon Species", Chemistry & Biodiversity, Bd. 5, Nr. 3, 1. März 2008, Seiten 461 bis 470) Untersuchungen zu ätherischen Ölen verschiedener Pflanzen im Hinblick auf ihre inhibitorischen Eigenschaften gegen SARS-CoV-2 und HSV-1.

Die internationale Patentanmeldung WO 2010/025821 A1 betrifft die Verwendung mindestens eines systemisch applizierbaren Monoterpens und mindestens eines topisch applizierbaren Atemwegstherapeutikums zur Behandlung von Atemwegserkrankungen, und zwar insbesondere bronchopulmonalen Erkrankungen. Hierbei fokussiert das Dokument auf Asthma bronchiale, Bronchitis, COPD sowie auf durch Tabakrauch induzierten chronischen Atemwegsentzündungen.

Nicht zuletzt betrifft die internationale Patentanmeldung WO 2021/216749 A1 flüssige pharmazeutische Flüssigzusammensetzungen, die oral verabreicht werden, und Verfahren zu ihrer Verwendung durch Verabreichung in die Lungen zur multifunktionalen Behandlung von Lungen- und Atemwegserkrankungen.

Vor diesem Hintergrund besteht eine Aufgabe der vorliegenden Erfindung darin, eine effiziente Therapie von durch Corona-Viren ausgelösten Viruserkrankungen bzw. von durch Corona-Viren ausgelösten viralen Infektionen (Virusinfektionen), insbesondere COVID-19, bereitzustellen.

Insbesondere ist eine Aufgabe der vorliegenden Erfindung darin zu sehen, einen geeigneten Wirkstoff und ein(e) diesen Wirkstoff enthaltendes Arzneimittel bzw. Zusammensetzung aufzufinden bzw. bereitzustellen, welcher bzw. welche(s) sich zur Verwendung bei der prophylaktischen und/oder therapeutischen Behandlung von durch Corona-Viren hervorgerufenen Viruserkrankungen bzw. von durch Corona-Viren hervorgerufenen viralen Infektionen (d. h. Corona(virus)infektionen), insbesondere COVID-19, eignet/eignen, bevorzugt im Rahmen einer effizienten Therapie.

In vollkommen überraschender Weise hat die Anmelderin nunmehr herausgefunden, dass sich 1 ,8-Cineol unerwartet und in effizienter Weise als Wirkstoff für die Therapie von durch Corona-Viren hervorgerufenen Viruserkrankungen bzw. von durch Corona-Viren hervorgerufenen viralen Infektionen (d. h. Corona(virus)infektionen), insbesondere COVID-19, eignet.

Zur Lösung der zuvor geschilderten Problemstellung schlägt daher die vorliegende Erfindung - gemäß einem **ersten** Aspekt der vorliegenden Erfindung -1 ,8-Cineol zur Verwendung bei der prophylaktischen und/oder therapeutischen Behandlung von durch Corona-Viren hervorgerufenen Viruserkrankungen (d. h. Corona-Virusinfektionen),nämlich COVID-19, gemäß Patentanspruch 1 vor. Jeweils vorteilhafte Weiterbildungen und Ausgestaltungen dieses Erfindungsaspekts sind Gegenstand der betreffenden Unteransprüche.

Ebenfalls Gegenstand der vorliegenden Erfindung - gemäß einem **zweiten** Aspekt der vorliegenden Erfindung - ist ein Arzneimittel oder Medikament zur Verwendung bei der prophylaktischen und/oder therapeutischen Behandlung von durch Corona-Viren hervorgerufenen Viruserkrankungen, nämlich COVID-19, insbesondere ein Antivirusmittel, gemäß Patenanspruch 12. Jeweils vorteilhafte Weiterbildungen und Ausgestaltungen dieses Erfindungsaspekts sind Gegenstand des betreffenden Unteranspruchs.

Gleichermaßen ist Gegenstand der vorliegenden Erfindung - gemäß einem **drit- ten** Aspekt der vorliegenden Erfindung - eine Zusammensetzung zur Verwendung bei der prophylaktischen und/oder therapeutischen Behandlung von durch Corona-Viren hervorgerufenen Viruserkrankungen, nämlich COVID-19, gemäß Patenanspruch 13. Jeweils vorteilhafte Weiterbildungen und Ausgestaltungen dieses Erfindungsaspekts sind Gegenstand des betreffenden Unteranspruchs.

Schließlich ist Gegenstand der vorliegenden Erfindung - gemäß einem **vierten** Aspekt der vorliegenden Erfindung - gleichermaßen eine pharmazeutische Kombination zur Verwendung bei der prophylaktischen und/oder therapeutischen Behandlung von durch Corona-Viren hervorgerufenen Viruserkrankungen,nämlich COVID-19, gemäß Patentanspruch 15.

Es versteht sich von selbst, dass Ausgestaltungen, Ausführungsformen, Vorteile und dergleichen, welche nachfolgend zu Zwecken der Vermeidung von Wiederholungen zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Darüber hinaus verhält es sich im Hinblick auf die nachfolgende Beschreibung der vorliegenden Erfindung auch derart, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von dem angegebenen Bereich bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Ferner ist bei allen nachstehend genannten relativen bzw. prozentualen, insbesondere gewichtsbezogenen Mengenangaben zu beachten, dass diese Angaben im Rahmen der erfindungsgemäßen Zusammensetzung vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Gegenstand der vorliegenden Erfindung - gemäß einem **ersten** Aspekt der vorliegenden Erfindung - ist somit 1 ,8-Cineol zur Verwendung bei der prophylaktischen und/oder therapeutischen Behandlung von COVID-19, wobei das 1,8-Cineol als magensaftresistente, aber dünndarmlösliche systemische Darreichungsform appliziert wird, wobei das 1,8-Cineol als Reinstoff vorliegt, wobei das 1,8-Cineol mit einer Reinheit von mindestens 95 Gew.-%, bezogen auf das 1,8-Cineol, vorliegt und wobei das 1,8-Cineol frei von anderen Terpenen ist.

Denn - wie zuvor ausgeführt - hat die Anmelderin in vollkommen überraschender Weise gefunden, dass 1,8-Cineol sich unerwartet und in effizienter Weise als Wirkstoff für die Therapie von durch Corona-Viren hervorgerufenen Viruserkrankungen bzw. von durch Corona-Viren hervorgerufenen viralen Infektionen (d. h. Corona(virus)infektionen)wie COVID-19 eignet.

Diese von der Anmelderin im Rahmen der vorliegenden Erfindung überraschend aufgefundene, spezielle und neue wie erfinderische Anwendung bzw. medizinische Indikation für 1,8-Cineol ist im Stand der Technik bislang nicht beschrieben und auch noch nicht einmal erkannt worden, obwohl es sich bei 1,8-Cineol an sich um einen hinlänglichen bekannten Wirkstoff handelt.

Bei dem erfindungsgemäß eingesetzten Wirkstoff 1,8-Cineol handelt es sich um ein sogenanntes Terpen, insbesondere Monoterpen. Bei Terpenen handelt es sich im Allgemeinen um Naturstoffe, welche als Bestandteil der sogenannten ätherischen Öle in Form von Flüssigkeiten aus Pflanzen oder deren Bestandteilen isoliert werden können. Sie sind oftmals Duft- und Geschmacksstoffe, welche im Bereich der Lebensmittelindustrie oder der Kosmetikindustrie Anwendung finden. Daneben gewinnt jedoch auch der Einsatz von Terpenen für medizinische Zwecke an Bedeutung, da sich für eine Vielzahl von Terpenen pharmakologische Wirkungen nachweisen lassen. Terpene stellen formal Polymerisationsprodukte des Isoprens dar, wobei nach der Anzahl der Isoprenreste zwischen Monoterpenen (C₁₀-Einheiten), Sesquiterpenen (C₁₅-Einheiten), Diterpenoiden (C₂₀-Einheiten), Sesterterpenen (C₂₅-Einheiten), Triterpenen (C₃₀-Einheiten), Tetraterpenen (C₄₀-Einheiten) und Polyterpenen unterschieden wird (vgl. RÖMPP-Chemielexikon, 10. Auflage, Georg Thieme Verlag, Stuttgart/New York, 1999, Seiten 4449 und 4450, Stichwort "Terpen(oid)e"). Darüber hinaus können Terpene auch als pharmakologische Wirksubstanzen Ausgangsstoffe für die Herstellung von Arzneimitteln oder Vitaminpräparaten sowie aufgrund ihrer oftmals bakterioziden bzw. pestiziden Wirkungen in der Landwirtschaft eingesetzt werden. Speziell für eine Anzahl von Monoterpenen sind pharmakologische Wirkungen in der Bekämpfung von Krankheiten bei erfolgter systemischen Behandlungen nachgewiesen, wobei insbesondere Menthol und 1,8-Cineol zu nennen sind.

Speziell der erfindungsgemäß eingesetzte Wirkstoff 1,8-Cineol gehört zu den bicyclischen Epoxy-Monoterpenen, genauer gesagt den Limonenoxiden. Synonyme Bezeichnungen für 1,8-Cineol mit der chemischen Summenformel C₁₀H₁₈O sind Eucalyptol, Limonen-1,8-oxid, 1,8-Epoxy-p-menthan oder 1,3,3-Trimethyl-2-oxab-icyclo[2.2.2]octan. Es handelt sich um eine farblose Flüssigkeit mit würzigem, campherähnlichem Geruch mit einem Schmelzpunkt von +1,5 C und mit einem Siedepunkt von 176 bis 177 °C, welche in Wasser unlöslich, aber mit den meisten organischen Lösemitteln mischbar ist.

Natürlich kommt 1,8-Cineol als Hauptbestandteil des Eukalyptusöls (Eukalyptusöl enthält bis zu 85 Gew.-% an 1,8-Cineol), aber auch in anderen Pflanzen vor, so z. B. in Minze, Heilsalbei, Thymian, Basilikum und im Teebaum. Darüber hinaus ist 1,8-Cineol beispielsweise auch in Niaouli-, Juniperus-, Piper-, Cannabis-, Kajeput-, Salbeiöl, Myrtenöl und anderen ätherischen Ölen enthalten.

Technisch bzw. pharmazeutisch aufgereinigtes 1,8-Cineol, welches im Allgemeinen mit 99,6 %-iger bis 99,8 %-iger Reinheit vorliegen kann, wird im Allgemeinen durch fraktionierte Destillation von Eukalyptusöl gewonnen.

1,8-Cineol wird im Stand der Technik insbesondere als Expektorans bei Bronchialkatarrhen und anderen Atemwegserkrankungen, aber darüber hinaus auch als Aromastoff in der Parfümindustrie angewendet. Darüber hinaus wird 1,8-Cineol in der Zahnmedizin bei der Revision von Wurzelfüllungen verwendet. In physiologischer Hinsicht wirkt 1,8-Cineol in den oberen und unteren Atemwegen, insbesondere in der Lunge und den Nebenhöhlen, schleimlösend. 1,8-Cineol wird nach dem Stand der Technik sowohl topisch (z. B. inhalativ) als auch systemisch (z. B. in Form von Kapseln), angewendet werden, meist als Mischöl zusammen mit einer Vielzahl weiterer Terpene.

Für weitergehende Einzelheiten zu dem Wirkstoff 1,8-Cineol kann beispielsweise verwiesen werden auf RÖMPP Chemielexikon, Georg Thieme Verlag, Stuttgart/New York, 10. Auflage, Band 1, 1996, Seite 752, Stichwort: "Cineol", sowie die dort referierte Literatur.

Vollkommen überraschend und unerwartet ist jedoch das von der Anmelderin im Rahmen der vorliegenden Erfindung aufgefundene antivirale Wirkpotential von 1,8-Cineol im Rahmen der erfindungsgemäßen Verwendung bei der prophylaktischen und/oder therapeutischen Behandlung von durch Corona-Viren hervorgerufenen Viruserkrankungen, nämlich COVID-19.

Wie die Anmelderin in diesem Zusammenhang überraschend gefunden hat, können Thrombozyten (Blutplättchen), welche neben der Blutgerinnung auch als immunregulatorische Entzündungszellen fungieren, Coronaviren beherbergen und somit durch Coronaviren ausgelöste Virusinfektionen, nämlich COVID-19, triggern, wobei das Cineol überraschenderweise insbesondere einem im Rahmen der Coronavirusinfektion auftretetenden Hyperinflammationssyndrom und vor allem einer Hyperzytokinämie (Zytokinsturm) entgegenwirken kann und die Coronavirusinfektion einzudämmen imstande ist.

Bei bisherigen COVID-19-Infektionsverläufen ist bekannt, dass insbesondere und grundsätzlich zwei Zustände der Entzündung eintreten können, nämlich derjenige, welcher zu einer Hyperinflammation mit potentiell kritischem Verlauf führt, oder derjenige, welcher zu einer viralen Clearance führt.

Vor allem sind Thrombozyten aus der Blutgerinnung und der Wahrung der vaskulären Integrität bekannt; aber jüngere Studien zeigen auch, dass Thrombozyten wichtige aktive Regulatoren des Immunsystems sind und insbesondere dem angeborenen Immunsystem zuzurechnen sind. In diesem Zusammenhang konnten die Bedeutung der Thrombozyten an entzündlich bedingten Erkrankungen nachgewiesen und auf diese Weise Einblicke in die Pathomechanismen erhalten werden.

Thrombozyten sind im Zusammenhang von viralen Infektionen insbesondere in der Lage, massive Entzündungsreaktionen (insbesondere eine Hyperzytokinämie) zu induzieren und aufrechtzuerhalten sowie Antigene zu präsentieren und Immunreaktionen in viralen Erkrankungen zu starten, aber auch RNA aufzunehmen und diese horizontal an andere Zellen weiterzugeben und darüber hinaus RNA-Viren aufzunehmen und ihnen einen Replikationsort zu bieten. Des Weiteren sind Thrombozyten imstande, sogenannte plättchen- bzw. thrombozytenderivierte Mikrovesikel (*plateletderived microparticles* bzw. *PMP*) zu produzieren und durch PMP-Immunzellen zu regulieren.

Aufgrund von klinischen Beobachtungen bei COVID-19-Infektionen können Thrombocyten als zentrale Zielzellen einer COVID-19-Infektion angesehen bzw. bewertet werden: So kann ein verändertes Thrombozyten/Lymphozyten-Verhältnis (sogenanntes *platelet-to-lymphocyte ratio* bzw. *PTR*) als inflammatorischer Marker und prognostischer Faktor in Bezug auf den Verlauf von COVID-19-Infektionen herangezogen werden. Auch ein verändertes Koagulationsverhalten lässt den Hinweis zu, dass PTL-Inhibition mit einem milderen Verlauf bei COVID-19-Infektionen verbunden ist. Eine Thrombozytopenie dagegen kann als Marker für einen schweren COVID-19-Verlauf gewertet werden. COVID-19-Infektionen sind im Allgemeinen durch eine Vielzahl proinflammatorische Effektor-Zytokine gekennzeichnet, welche aus Thrombozyten freigesetzt werden, wie z. B. TNF, IL-1β, IL-6, IL-8, G-CSF und GM-CSF.

Im Rahmen der vorliegenden Erfindung konnte durch die Anmelderin überraschend ein grundlegendes klinisches und immunologisches Verständnis in Bezug auf die Alteration von Thrombocyten im Kontext von COVID-19-Infektionen unter Cineolbehandlung gewonnen werden.

In diesem Zusammenhang konnte seitens der Anmelderin gezeigt werden, dass 1,8-Cineol effizient in der Behandlung von durch Corona-Viren hervorgerufenen Viruserkrankungen, nämlich COVID-19, eingesetzt werden kann.

So konnte beispielsweise bei *in*-*vitro*-Studien mit aus gesunden Probanden gewonnenen Thrombocyten, welche zunächst mit 1,8-Cineol inkubiert und dann mit Corona-Viren infiziert wurden, gezeigt werden, dass sich ein Infektionsausbruch, insbesondere eine Hyperzytokinämie, präventiv verhindern bzw. vermindern lässt. In weiteren *in*-*vitro*-Studien mit aus an COVID-19 erkrankten Patienten gewonnenen Thrombocyten, welche mit 1,8-Cineol inkubiert wurden, ließen sich diese Ergebnisse bestätigen, insbesondere dahingehend, dass sich der Infektionsverlauf, insbesondere eine Hyperzytokinämie, verhindern bzw. abschwächen lässt. Diese antivirale Wirkung von 1,8-Cineol bei COVID-19-Infektionen ist vollkommen unerwartet.

In diesem Zusammenhang ist es bekannt, dass Thrombozyten an Lungenepithelien adhärieren und auf diese Weise eine Infektion der Epithelien mit COVID-19 ermöglichen bzw. exazerbieren. Die Adhäsion an Epithelien ist wiederum zytokinabhängig und kann folglich durch 1,8-Cineol inhibiert bzw. verringert oder abgeschwächt werden. Damit wird auch eine Infektion der Epithelien und letztlich des Lungengewebes abgemildert bzw. verhindert.

Dieser von der Anmelderin vollkommen überraschend aufgefundene Effekt lässt sich - ohne sich auf eine bestimmte Theorie festlegen zu wollen - möglicherweise unter anderem auch darauf zurückführen, dass eine systemische, insbesondere perorale, Verabreichung von 1,8-Cineol dazu führt, dass insbesondere infolge von Diffusions- und/oder Exhalationsprozessen ein gewisser Teil des systemisch bzw. peroral applizierten 1,8-Cineols unverändert in das Atemwegsepithel, insbesondere in das Alveolar- und/oder Bronchialepithel, gelangt und dort sozusagen ausgeatmet wird und sich infolgedessen in relativ hohen Konzentrationen auf und in den Epithelzellen wiederfindet, so dass es dort in effizienter Weise seine Wirkung entfalten kann. Dies ist im Rahmen einer erfindungsgemäßen Therapie von durch Corona-Viren verursachten viralen Infektionen (Virusinfektionen), nämlich COVID-19, mit 1,8-Cineol von besonderem Vorteil, insbesondere da bei einer Vielzahl der Infektionen eine Entzündung des Lungenepithels auftritt.

Infolge der Lipophilie des systemisch bzw. peroral applizierten 1,8-Cineols, wird zudem eine Langzeitspeicherung in den betreffenden Epithelzellen beobachtet, so dass auch eine langfristige und gleichmäßige Versorgung mit dem 1,8-Cineol in den betreffenden Epithelzellen gewährleistet ist. Auch dies ist in Bezug auf eine erfindungsgemäße Behandlung von durch Corona-Viren verursachten viralen Infektionen (Virusinfektionen), nämlich COVID-19, besonders vorteilhaft.

Die Wirksamkeit des erfindungsgemäß bei der Behandlung von durch Corona-Viren verursachten viralen Infektionen (Virusinfektionen), nämlich COVID-19, eingesetzten 1,8-Cineols lässt sich - wiederum ohne sich auf eine spezielle Theorie festlegen zu wollen - möglicherweise auch auf das von der Anmelderin überraschend aufgefundene steroidartige Wirkungspotential des eingesetzten 1,8-Cineols erklären, insbesondere im Hinblick auf die Hemmung von Entzündungsmediatoren, d. h. 1,8-Cineol, insbesondere als Reinsubstanz, besitzt somit ein steroidartiges Wirkungspotential und ist imstande, die Hemmung von Entzündungsmediatoren zu bewirken. Ätherische Mischöle dagegen (welche Cineol in Gemisch mit weiteren Terpenen und anderen Wirkstoffen enthalten) stimulieren die Prostaglandinproduktion und zeigen eine gegenüber reinem 1,8-Cineol nur verminderte Hemmung der Leukotrien- und Zytokinproduktion; denn derartige Mischöle enthalten auch solche Substanzen, welche die Zellaktivität und die Mediatorproduktion stimulieren und daher nicht entzündungshemmend wirken, sondern Unverträglichkeitsreaktionen verursachen können, so dass ätherische Mischöle bzw. Ölgemische folglich im Allgemeinen sogar die Zellaktivität erhöhen und eine Entzündungsmediatorproduktion induzieren können. Im Unterschied hierzu jedoch bewirkt 1,8-Cineol, insbesondere in Reinform, eine signifikante Hemmung der Mediatorproduktion; hierdurch wird somit ein entzündungshemmender Effekt bewirkt. Dieser Effekt wird bei systemischer Applikation von 1,8-Cineol im gesamten Körper und vor allem in den gesamten Atemwegen, insbesondere in der gesamten Lunge, d. h. auch bis in die Peripherie und die Alveolen, erreicht. Denn 1,8-Cineol wird nach systemischer Gabe (insbesondere in Form von magensaftresistenten, aber dünndarmlöslichen Kapseln) ins Blut aufgenommen und entsprechend seinen physikalischen Eigenschaften unter anderem auch in den Alveolen freigesetzt. Hierdurch kann das 1,8-Cineol im gesamten Körper und vor allem auch in den kleinsten, peripher gelegenen Atemwegen eine entzündungshemmende Wirkung vermitteln.

Aufgrund dieses Wirkprofils eignet sich 1,8-Cineol bei der prophylaktischen und/oder therapeutischen Behandlung von durch Corona-Viren hervorgerufenen Viruserkrankungen, nämlich COVID-19, vor allem als Co-Therapeutikum zu Kortikosteroiden, insbesondere Dexamethason: Wie die Anmelderin überraschend gefunden hat, ist 1,8-Cineol auch imstande, die Wirksamkeit bzw. Effizienz von bei der Behandlung von durch Corona-Viren hervorgerufenen Viruserkrankungen, nämlich COVID-19, insbesondere systemisch verabreichten Kortikosteroiden (z. B. insbesondere Dexamethason) signifikant, insbesondere in synergistischer Weise, zu steigern und deren eingesetzte Dosismengen signifikant zu reduzieren, verbunden mit den damit einhergehenden Vorteilen (z. B. Vermeidung bzw. Reduzierung von Nebenwirkungen etc.). Gleichzeitig wird durch das Zusammenwirken von 1,8-Cineol einerseits und von insbesondere systemisch verabreichten Kortikosteroiden (z. B. insbesondere Dexamethason) andererseits die Sensitivität gegenüber den insbesondere systemisch verabreichten Kortikosteroiden (z. B. insbesondere Dexamethason) signifikant gesteigert; folglich kann gegebenenfalls die Dosismenge der insbesondere systemisch verabreichten Kortikosteroide (z. B. insbesondere Dexamethason) reduziert werden.

Durch Untersuchungen seitens der Anmelderin wurde überraschend gefunden, dass 1,8-Cineol die Wirkung von Kortikosteroiden (z. B. insbesondere Dexamethason) in signifikanter, insbesondere synergistischer Weise, zu steigern imstande ist. Es wurde überraschend gefunden, dass therapeutisch relevante Konzentrationen von 1,8-Cineol zusammen mit Kortikosteroiden (z. B. insbesondere Dexamethason) eine synergistische Hemmung der Zytokinproduktion bewirken und folglich die antiinflammatorische Wirkung von Kortikosteroiden signifikant verstärken können.

Wie die Anmelderin im Rahmen von *in*-*vitro*-Studien an humanen Monozytenkulturen zeigen konnte, bewirkt bereits 1,8-Cineol allein, dass die LPS-stimulierte Produktion von IL-1beta signifikant gehemmt wird. In Kombination mit einem Kortikosteroid (z. B. insbesondere Dexamethason) kann 1,8-Cineol den durch das Kortikosteroid gleichermaßen hervorgerufenen Effekt in synergistischer Weise steigern.

Weiterführende Studien der Anmelderin zeigen zudem, dass selbst niedrige therapeutische Konzentrationen von 1,8-Cineol die Produktion von IL-1beta (Interleukin-1beta) im Vergleich zu subtherapeutischen Konzentrationen von Kortikosteroiden (z. B. insbesondere Dexamethason), für welche keine signifikante Hemmung nachgewiesen werden, dennoch signifikant hemmen können, d. h. durch Kombination von 1,8-Cineol mit den betreffenden Kortikosteroiden (z. B. insbesondere Dexamethason) kann eine signifikante Hemmung sogar für subtherapeutische, aber selbstverständlich auch für therapeutische Dosen bzw. Konzentrationen von Kortikosteroiden (z. B. insbesondere Dexamethason) mit einer resultierenden Intensivierung der Wirkung der Kortikosteroide nachgewiesen werden.

Aber auch bereits 1,8-Cineol allein bewirkt gemäß den *in*-*vitro*-Studien der Anmelderin eine signifikante Hemmwirkung in Bezug auf die Produktion von Zytokinen, insbesondere der Zytokine IL-8 (Interleukin-8) und TNF-alpha (Tumornekrosefaktoralpha), in humanen Monozyten von Probanden.

Die Studien der Anmelderin zeigen somit insgesamt, dass der Wirkstoff 1,8-Cineol vor allem in Form der Reinsubstanz, imstande ist, bei der prophylaktischen und/oder therapeutischen Behandlung von durch Corona-Viren hervorgerufenen Viruserkrankungen, nämlich COVID-19, den betreffenden Infektionsverlauf abzuschwächen bzw. abzuwehren, insbesondere eine Hyperzytokinämie zu verhindern bzw. abzuschwächen. Diese antivirale Wirkung von 1,8-Cineol bei COVID-19-Infektionen ist vollkommen unerwartet und lässt sich durch systemische Gabe von Kortikosteroiden (z. B. insbesondere Dexamethason) sogar noch weiterführend bzw. steigern, insbesondere in synergistischer Weise.

Weiterhin wirkt - wie die Anmelderin gleichermaßen überraschend herausgefunden hat - das erfindungsgemäß eingesetzte 1,8-Cineol auch als Antioxidans und NO-Regulator in Bezug auf bei durch Corona-Viren hervorgerufenen Viruserkrankungen, nämlich COVID-19, auftretende Oxidationsprozesse und NO-Mangelzustände im Körper bzw. in den Organen der betroffenen Patienten.

Daher kann im Rahmen der erfindungsgemäßen Verwendung das 1,8-Cineol, insbesondere auch als Induktor der NO-Produktion für die bzw. im Rahmen der Behandlung von durch Corona-Viren hervorgerufenen Viruserkrankungen, nämlich COVID-19, eingesetzt werden.

Des Weiteren kann das 1,8-Cineol im Rahmen der erfindungsgemäßen Verwendung insbesondere auch zur Verbesserung der Gewebs- und/oder Mikroperfusion bei NO-Mangelsituationen in Bezug auf die betroffenen Organe eingesetzt werden.

Denn NO als solches ist grundsätzlich bekannt als antiinflammatorischer Mediator sowie als Hemmstoff von Entzündungsmediatoren und der Thrombozytenaggregation und als Aktivator der ziliären Funktion und mukosalen Clearance und schützt insbesondere vor respiratorischen Infekten und Exazerbationen respiratorischer Infekte. In dieser Hinsicht ist 1,8-Cineol folglich geeignet als Wirkstoff bzw. Therapeutikum, welcher bzw. welches die bei Coronavirus-Infektionen vorliegende supprimierte NO-Produktion durch günstigen Abbau von O₂⁻-Radikalen mit Induktion von NO normalisiert und den jeweiligen Erfordernissen entsprechend durch Modulation adäquat anpasst. Daraus resultiert im Rahmen der vorliegende Erfindung eine vollkommen neue Indikation für den Einsatz von 1,8-Cineol (z. B. bevorzugt in einer höheren, systemisch wirksamen Tagesdosis, beispielsweise von z. B. 600 bis 1.200 mg, insbesondere zur Regulation der Organperfusion und insbesondere auch zum Schutz oberer und unterer Atemwege, einschließlich der Lunge).

Im Rahmen der vorliegenden Erfindung wurde auf der Suche nach den zugrundeliegenden Eigenschaften von systemisch verabreichtem 1,8-Cineol, insbesondere zur Intensivierung antiinflammatorischer Wirkungen, zudem vollkommen überraschend auch eine antioxidative Wirkung für 1,8-Cineol, insbesondere infolge der Hemmung der Produktion von Superoxiden (O₂⁻-Radikale), der Aktivität von Superoxiddismutasen (SOD) und von Wasserstoffperoxid (H₂O₂), welches als Endprodukt der Oxidation die Produktion von Entzündungsmediatoren, insbesondere von Zytokinen und Arachidonsäuremetaboliten, stimuliert, aufgefunden. Hierbei wurde überraschend auch eine Hemmung der Spontanproduktion von O₂⁻-Radikalen bei therapeutischen Konzentrationen von 1,8-Cineol nachgewiesen, wobei das 1,8-Cineol bereits bei niedrigeren Konzentrationen im therapeutischen Bereich die Produktion von O₂⁻-Radikalen und H₂O₂ hemmt. Als Ursache für diese antioxidativen Wirkungen von 1,8-Cineol wurde überraschenderweise die Wirkung von 1,8-Cineol als aktiver Induktor der NO-Produktion gefunden. Somit wurde gefunden, dass 1,8-Cineol aktiv die NO-Produktion durch Vermittlung antioxidativer Wirkungen induziert. Hierbei konnten von der Anmelderin somit modulierende Einflüsse von 1,8-Cineol zur Kontrolle oxidativer und somit zellschädigender wie proinflammatorischer Einflüsse durch Hemmung von O₂⁻-Radikalen und eine gegenläufige Stimulation von antiinflammatorischem NO im therapeutischen Dosisbereich von 1,8-Cineol nachgewiesen werden. Insgesamt eignet sich somit 1,8-Cineol auf Basis der überraschend aufgefundenen Erkenntnisse der Anmelderin in effizienter Weise zur Verwendung bei der prophylaktischen und/oder therapeutischen Behandlung von COVID-19 (z. B. als alleiniges Therapeutikum oder aber in Co-Therapie mit weiteren Wirkstoffen, wie zuvor beschrieben, insbesondere Kortikosteroiden, wie Dexamethason).

Wie zuvor ausgeführt, ist Gegenstand der vorliegenden Erfindung - gemäß einem ersten Erfindungsaspekt - 1,8-Cineol zur Verwendung bei der prophylaktischen und/oder therapeutischen Behandlung von COVID-19, wobei das 1,8-Cineol als magensaftresistente, aber dünndarmlösliche systemische Darreichungsform appliziert wird, wobei das 1,8-Cineol als Reinstoff vorliegt, wobei das 1,8-Cineol mit einer Reinheit von mindestens 95 Gew.-%, bezogen auf das 1,8-Cineol, vorliegt und wobei das 1,8-Cineol frei von anderen Terpenen ist.

Gemäß einer besonderen Ausführungsform der vorliegenden Erfindung gemäß diesem Erfindungsaspekt ist es vorgesehen, dass das 1,8-Cineol systemisch, insbesondere peroral, appliziert wird und/oder dass das 1,8-Cineol zur systemischen, insbesondere peroralen, Applikation hergerichtet ist. Auf diese Weise werden hohe systemische Wirkdosen bzw. Wirkstoffpegel (d. h. Wirkstoffspiegel bzw. -konzentrationen) erreicht, so dass eine besonders gute Wirksamkeit bzw. Effizienz realisiert werden kann.

Gemäß einer vorteilhaften Ausführungsform nach diesem Erfindungsaspekt ist es insbesondere vorgesehen, dass das 1,8-Cineol in Form einer peroral zu verabreichenden Darreichungsform appliziert wird und/oder dass das 1,8-Cineol in einer peroral zu verabreichenden Darreichungsform hergerichtet ist.

Gemäß der vorliegenden Erfindung gemäß diesem Aspekt ist es vorgesehen, dass das 1,8-Cineol als magensaftresistente, aber dünndarmlösliche systemische Darreichungsform, bevorzugt als Kapsel, Dragee, Pille, Tablette oder dergleichen, appliziert wird und/oder kann das 1,8-Cineol zur Verabreichung als magensaftresistente, aber dünndarmlösliche systemische Darreichungsform, bevorzugt Kapsel, Dragee, Pille, Tablette oder dergleichen, hergerichtet sein.

Für die systemische Anwendung von 1,8-Cineol sind im Handel verschiedene Präparate, insbesondere auf Basis von im Allgemeinen magensaftresistenten, aber dünndarmlöslichen Darreichungsformen bzw. Kapseln, verfügbar.

Der Vorteil derartiger Darreichungsformen ist, dass zum einen der Wirkstoff 1,8-Cineol langzeitstabil vorliegt und zum anderen besonders hohe systemische Wirkstoffkonzentrationen bzw. Wirkstoffpegel erreicht werden können. Auf diese Weise können auch besonders hochdosierte Darreichungsformen bereitgestellt werden, so dass eine genaue und hohe Dosierung des Wirkstoffs ermöglicht wird.

Im Rahmen der vorliegenden Erfindung wird das 1,8-Cineol üblicherweise in einer peroralen Darreichungsform, vorzugsweise in Form von Kapseln, eingesetzt. Erfindungsgemäß besonders bevorzugt ist die Applikation des des 1,8-Cineols in Form einer peroralen, magensaftresistenten, aber dünndarmlöslichen Darreichung, insbesondere Kapsel. Ganz besonders bevorzugt sind perorale, magensaftresistente, aber dünndarmlösliche Darreichungsformen, insbesondere Kapseln, welche 1,8-Cineol als Reinstoff enthalten. Derartige Produkte sind im Handel erhältlich bzw. verfügbar (z. B. Soledum^{®}-Kapseln, vertrieben von der Cassella-med GmbH & Co. KG bzw. der Maria Clementine Martin Klosterfrau Vertriebsgesellschaft mbH, jeweils Köln, Deutschland).

Im Allgemeinen ist es im Rahmen der vorliegenden Erfindung vorgesehen, dass die perorale Darreichungsform, insbesondere Kapsel, magensaftresistent, aber dünndarmlöslich ausgebildet ist. Hierdurch wird ein besonders optimales Freisetzungsprofil erreicht, da der Wirkstoff ziel- und zweckgerichtet erst im Darm freigesetzt wird.

Die im Rahmen dieser Erfindung verwendeten Definition für die Begriffe "magensaftresistent" bzw. "dünndarmlöslich" sowie die diesbezüglichen Testmethoden sind in *European Pharmacopoeia* 7.0, 04/2010: 1502, Seiten 707 bis 709, Stichwort "Cap*sules*", Unterkapitel "*Gastro-Resistant Capsules*" sowie *European Pharmacopoeia* 7.1, 04/2011: 20901, Seiten 3331 und 3332, Stichwort "2.9.1 *Disintegration of Tablets and Capsules*" wiedergegeben.

Unter dem Begriff "magensaftresistent" ist dabei im Rahmen der vorliegenden Erfindung insbesondere zu verstehen, dass die Kapseln mindestens zwei Stunden lang in 0,1 N Salzsäure, welche auf Temperaturen von 35 bis 39 °C erwärmt wird, unter ständiger Durchmischung aufbewahrt, insbesondere gerührt werden kann, ohne dass die Kapseln Anzeichen einer Zersetzung bzw. Risse oder andere Beschädigungen aufweisen.

Unter dem Begriff "dünndarmlöslich" ist im Rahmen der vorliegenden Erfindung hingegen insbesondere zu verstehen, dass die Kapseln in einer wässrigen Phosphatpuffer-Lösung, welche auf einen pH-Wert von etwa 6,8 eingestellt, unter Rühren bei Temperaturen im Bereich von 35 bis 39 °C innerhalb einer Stunde soweit zersetzt werden, dass die Wirksubstanz freigesetzt wird.

Im Rahmen der vorliegenden Erfindung ist es vorgesehen, dass das 1,8-Cineol in pharmazeutisch wirksamen bzw. therapeutisch wirksamen Mengen verabreicht wird und/oder dass das 1,8-Cineol zur Verabreichung in pharmazeutisch wirksamen bzw. therapeutisch wirksamen Mengen hergerichtet ist.

Insbesondere kann es im Rahmen der vorliegenden Erfindung gemäß diesem Erfindungsaspekt nach einer besonderen Ausführungsform vorgesehen sein, dass das 1,8-Cineol mit einer Tagesdosis im Bereich von 1 bis 5.000 mg/diem, insbesondere im Bereich von 2 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, verabreicht wird und/oder dass das 1,8-Cineol zur Verabreichung mit einer Tagesdosis im Bereich von 1 bis 5.000 mg/diem, insbesondere im Bereich von 2 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, hergerichtet ist.

Gemäß der vorliegenden Erfindung nach diesem Erfindungsaspekt ist es vorgesehen, dass das 1,8-Cineol als Reinstoff vorliegt und/oder verabreicht wird. Das 1,8-Cineol liegt dabei mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das 1,8-Cineol vor und/oder wird entsprechend verabreicht. Dabei ist das 1,8-Cineol frei von anderen Terpenen und/oder enthält das 1,8-Cineol keine anderen Terpene.

Mit anderen Worten ist es im Rahmen der vorliegenden Erfindung gemäß diesem Erfindungsaspekt vorgesehen, dass das 1,8-Cineol in Abwesenheit von anderen (weiteren) Terpenen vorliegt und/oder verabreicht wird.

Gemäß der erfindungsgemäßen Ausführung ist es also vorgesehen, dass das 1,8-Cineol als Reinstoff und/oder in Abwesenheit von anderen (weiteren) Terpenen vorliegt und/oder verabreicht wird.

Wie vorstehend geschildert, ist es also erfindungsgemäß vorgesehen, dass die monoterpenbasierte Wirksubstanz (d. h. 1,8-Cineol) in Form einer Reinsubstanz, d. h. als alleiniger bzw. technisch isolierter/aufgereinigter Wirkstoff, eingesetzt wird. Auf diese Weise kann beispielsweise eine immer gleichbleibende Dosierung bzw. ein immer gleicher Gehalt an Wirksubstanz in der erfindungsgemäß vorgesehen Darreichungsform gewährleistet werden. Auch sind auf diese Weise hohe systemische Wirkstoffdosen bzw. -pegel realisierbar.

Vor allem besitzt - wie zuvor erläutert - 1,8-Cineol in Reinform bzw. als Reinstoff ein besonders effizientes steroidartiges Wirkungspotential, da es in Reinform bzw. als Reinstoff nämlich eine besonders effiziente Hemmung von Entzündungsmediatoren bzw. der Leukotrien- und Zytokinproduktion bewirkt. Ätherische Mischöle, welche Cineol als eine von vielen Wirkkomponenten enthalten (wie z. B. Eukalyptusöl etc.) dagegen stimulieren die Prostaglandinproduktion und zeigen nur eine gegenüber reinem Cineol signifikant verminderte Hemmung der Leukotrien- und Zytokinproduktion, da die Mischöle auch solche Substanzen enthalten, welche die Zellaktivität und die Mediatorproduktion stimulieren können und daher nicht entzündungshemmend wirken, sondern vielmehr Unverträglichkeitsreaktionen verursachen können (d. h. derartige ätherische Mischöle bzw. Ölgemische können folglich im Allgemeinen die Zellaktivität erhöhen und sogar eine Entzündungsmediatorproduktion induzieren). Im Unterschied hierzu jedoch bewirkt isoliertes bzw. reines 1,8-Cineol ausschließlich eine signifikante Hemmung der Entzündungsmediatorproduktion.

Entsprechend vorgesehen ist es, dass das 1,8-Cineol in Reinform bzw. als Reinstoff und systemisch verabreicht wird. Denn das 1,8-Cineol in Reinform bzw. als Reinstoff wird nach systemischer Gabe, insbesondere in Form von magensaftresistenten, aber dünndarmlöslichen Kapseln, ins Blut aufgenommen und entsprechend seinen physikalischen Eigenschaften auch in hohen Wirkstoffdosen bzw. Wirkstoffkonzentrationen in den Alveolen freigesetzt; hierdurch kann das 1,8-Cineol gerade auch in den kleinsten, peripher gelegenen Atemwegen eine entzündungshemmende Wirkung im Rahmen einer Therapie von durch Corona-Viren hervorgerufenen viralen Infektionen bzw. Viruserkrankungen wie COVID-19 vermitteln.

Gemäß einer wiederum weiteren besonderen Ausführungsform gemäß diesem Erfindungsaspekt kann es erfindungsgemäß vorgesehen sein, dass das 1,8-Cineol zusammen mit mindestens einem physiologisch unbedenklichen Träger (Exzipienten), vorliegt und/oder verabreicht wird. Insbesondere kann es dabei vorgesehen sein, dass der physiologisch unbedenkliche Träger (Exzipient) mit 1,8-Cineol mischbar und/oder hierin löslich ist. Weiterhin kann es insbesondere dabei vorgesehen sein, dass der physiologisch unbedenkliche Träger (Exzipient) bei 20 °C und bei Atmosphärendruck im flüssigen oder festen, vorzugsweise flüssigen, Aggregatzustand vorliegt. Wiederum weiterhin kann es in diesem Zusammenhang vorgesehen sein, dass der physiologisch unbedenkliche Träger (Exzipient) ausgewählt sein kann aus der Gruppe von Fettsäureestern, bevorzugt Triglyceriden von Fettsäuren, besonders bevorzugt mittelkettigen Triglyceriden (*Medium Chain Triglycerides* bzw. MCT), ganz besonders bevorzugt Triglyceriden von C₆-C₁₂-Fettsäuren.

Gemäß einer besonderen Ausführungsform enthält somit - wie zuvor geschildert - die erfindungsgemäß vorgesehene Darreichungsform das Monoterpen und Wirkstoff 1,8-Cineol zusammen mit mindestens einem mit der Wirksubstanz mischbaren und/oder hierin löslichen, insbesondere bei 20 °C und Atmosphärendruck flüssigen, physiologisch unbedenklichen Träger (Exzipienten). Der Träger bzw. Exzipient sollte selbst pharmakologisch nicht wirksam sein, aber mit der Wirksubstanz eine vorzugsweise homogene Mischung oder Lösung bilden.

Gemäß einer erfindungsgemäß bevorzugten Ausführungsform kann also dabei, wie zuvor erläutert, der Träger bzw. Exzipient ausgewählt sein aus der Gruppe von Fettsäureestern, bevorzugt Triglyceriden von Fettsäuren, besonders bevorzugt mittelkettigen Triglyceriden (*Medium Chain Triglycerides* bzw. MCT), ganz besonders bevorzugt Triglyceriden von C₆-C₁₂-Fettsäuren.

Als Träger bzw. Exzipient bevorzugt eingesetzt werden können insbesondere Gemische von Fettsäuretriglyceriden, welche bei Raumtemperatur (insbesondere 20 °C) und Atmosphärendruck flüssig sind; insbesondere bezeichnet dieser Begriff Ester des dreiwertigen Alkohols Glycerin (Propan-1,2-3-triol) mit drei, meist verschiedenen, überwiegend geradzahligen und unverzweigten aliphatischen Monocarbonsäuren, den sogenannten Fettsäuren. Verbindungen dieser Art werden auch Triglyceride genannt (nach IUPAC-Empfehlung: Triacylglycerine). Triglyceride, synonym auch als Glycerol-Triester bezeichnet, sind also dreifache Ester des dreiwertigen Alkohols Glycerin mit drei Säuremolekülen, wobei die Vorsilbe "*tri*" auf drei Acyl-Säurereste, die mit Glycerin verestert sind, verweist.

Speziell mittelkettige Triglyceride, wie sie erfindungsgemäß bevorzugt als Träger bzw. Exzipient für die monoterpenhaltige Wirksubstanz zum Einsatz kommen, sind insbesondere halbsynthetische neutrale Glycerinester von gesättigten, im Allgemeinen unverzweigten Monocarbonsäuren mittlerer Kettenlänge (d. h. C₆-C₁₂-Ketten). Insbesondere bezeichnet der Begriff der mittelkettigen Triglyceride Gemische von Triglyceriden gesättigter Fettsäuren, hauptsächlich Caprylsäure (Octansäure) und Caprinsäure (Decansäure). Mittelkettige Triglyceride können im Allgemeinen aus Öl hergestellt werden, welches aus dem festen und getrockneten Teil des Endosperms von *Cocos nucifera* L. und/oder aus dem getrockneten Endosperm von *Elaeis guineenses* Jacq. extrahiert wird. Für weitergehende Einzelheiten zu dem Begriff der mittelkettigen Triglyceride kann beispielsweise verwiesen werden auf die Monographie Ph. Eur., 6. Ausgabe, Grundwerk 2008, Seiten 4224 bis 4226, sowie auf die Zeitschrift für Ernährungswissenschaft, Band 13, Heft 1/2, 1973, Seiten 6 ff., D. Sailer et al. "Mittelkettige Triglyceride - Klinische Physiologie und Anwendung")*.*

Erfindungsgemäß bevorzugt ist es, wenn der Träger bzw. Exzipient in einem Wirksubstanz/Träger-Mengenverhältnis im Bereich von 1.000 : 1 bis 1 : 1.000, insbesondere 100 : 1 bis 1 : 100, vorzugsweise 50 : 1 bis 1 : 50, besonders bevorzugt 10 : 1 bis 1 : 10, ganz besonders bevorzugt 5 : 1 bis 1 : 2, noch mehr bevorzugt 3 : 1 bis 1 : 1, eingesetzt wird. Durch die Mischung der Wirksubstanz (d. h. 1,8-Cineol) mit dem Exzipienten werden eine deutlich verbesserte Kompatibilität der Wirksubstanz mit den weiteren Inhaltsstoffen der Darreichungsform und eine verbesserte Stabilität, insbesondere Lagerstabilität, erreicht.

Gemäß einer gleichermaßen weiteren besonderen Ausführungsform der vorliegenden Erfindung kann es vorgesehen sein, dass das 1,8-Cineol in bzw. in Form einer Zusammensetzung, insbesondere pharmazeutischen Zusammensetzung, insbesondere zusammen mit mindestens einem pharmazeutisch verträglichen und/oder physiologisch unbedenklichen Exzipienten (Träger), vorliegt und/oder verabreicht wird.

In diesem Zusammenhang kann es insbesondere vorgesehen sein, dass die Zusammensetzung das 1,8-Cineol als alleinigen Wirkstoff, insbesondere als alleinigen pharmazeutischen Wirkstoff, enthält.

Weiterhin kann es dabei insbesondere vorgesehen sein, dass die Zusammensetzung das 1,8-Cineol als Reinstoff enthält, vorzugsweise mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das 1,8-Cineol und/oder vorzugsweise frei von anderen Terpenen.

Gleichermaßen kann es dabei auch vorgesehen sein, dass die Zusammensetzung das 1,8-Cineol frei von anderen Terpenen enthält und/oder dass die Zusammensetzung kein weiteres Terpen enthält und/oder dass die Zusammensetzung frei von anderen Terpenen als 1,8-Cineol ist bzw. ausgebildet ist.

Weiterhin kann es dabei vorgesehen sein, dass die Zusammensetzung das 1,8-Cineol in wirksamen, insbesondere pharmazeutisch und/oder therapeutisch wirksamen, Mengen enthält.

Ebenfalls kann es in diesem Kontext erfindungsgemäß vorgesehen sein, dass die Zusammensetzung das 1,8-Cineol bezogen auf die Zusammensetzung, in relativen Mengen im Bereich von 0,0001 bis 80 Gew.-%, insbesondere 0,001 bis 75 Gew.-%, vorzugsweise 0,005 bis 70 Gew.-%, bevorzugt 0,01 bis 60 Gew.-%, besonders bevorzugt 0,05 bis 55 Gew.-%, ganz besonders bevorzugt 0,1 bis 50 Gew.-%, enthält.

Schließlich kann es erfindungsgemäß in diesem Zusammenhang auch vorgesehen sein, dass der mindestens eine pharmazeutisch verträgliche und/oder physiologisch unbedenkliche Exzipient (Träger) mit 1,8-Cineol mischbar und/oder hierin löslich ist. Vorzugsweise kann der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegen. Ebenfalls bevorzugt kann der mindestens eine pharmazeutisch verträgliche und/oder physiologisch unbedenkliche Exzipient (Träger) ausgewählt sein aus der Gruppe von Fettsäureestern, bevorzugt Triglyceriden von Fettsäuren, besonders bevorzugt mittelkettigen Triglyceriden (*Medium Chain Triglycerides* bzw. MCT), ganz besonders bevorzugt Triglyceriden von C₆-C₁₂-Fettsäuren.

Gemäß einer besonderen Ausführungsform der vorliegenden Erfindung kann das 1,8-Cineol in liposomenumgebener und/oder liposomal verpackter Form vorliegen und/oder verabreicht werden. Hierdurch ist ein besonders gutes Freisetzungsprofil gewährleistet.

Gemäß einer weiteren besonderen Ausführungsform der vorliegenden Erfindung kann das 1,8-Cineol in micellierter Form und/oder in einer micellaren Darreichungsform vorliegen und/oder verabreicht werden. Auch hierdurch kann ein besonders gutes Freisetzungsprofil gewährleistet werden.

Weiterhin kann es gemäß einer besonderen Ausführungsform vorgesehen sein, dass das Cineol ohne und/oder in Abwesenheit von nichtsteroidalen Entzündungshemmern (NSAID) vorliegt bzw. eingesetzt und/oder verabreicht wird.

Gemäß einer wiederum besonderen Ausführungsform der vorliegenden Erfindung gemäß diesem Erfindungsaspekt kann es vorgesehen sein, dass das 1,8-Cineol zusammen mit mindestens einem weiteren Wirkstoff angewendet oder appliziert wird. In diesem Zusammenhang kann es insbesondere vorgesehen sein, dass der weitere Wirkstoff ausgewählt ist aus (i) entzündungshemmenden Wirkstoffen, insbesondere Kortikosteroiden, (ii) blutverdünnenden oder blutgerinnungshemmenden Wirkstoffen, (iiii) antiviral wirkenden Wirkstoffen, insbesondere synthetischen antiviralen Wirkstoffen, wie Remdesivir, oder proteinbasierten antiviralen Wirkstoffen, wie insbesondere monoklonalen Antikörpern, Immunglobulinen und Interferonen; (iv) kardiovaskulären Wirkstoffen, insbesondere Blutdrucksenkern und ACE-Hemmern; sowie deren Kombinationen.

Gemäß einer besonders bevorzugten Ausführungsform kann es dabei insbesondere vorgesehen sein, dass der weitere Wirkstoff getrennt, insbesondere räumlich getrennt, von dem 1,8-Cineol aber funktional zusammenhängend hiermit eingesetzt und/oder verabreicht wird, insbesondere in Form eines Kit (Kit-of-part).

Auf die vorgenannte Art und Weise (d. h. Kombination oder gemeinsame Verabreichung mit mindestens einem weiteren Wirkstoff) gelingt es insbesondere, die Wirkung bzw. Effizienz des weiteren Wirkstoffs, insbesondere im Fall von Kortikosteroiden (wie z. B. Dexamethason), signifikant, insbesondere in synergistischer Weise, zu steigern und deren eingesetzte Dosismengen signifikant zu reduzieren, verbunden mit den damit einhergehenden Vorteilen (z. B. Vermeidung bzw. Reduzierung von Nebenwirkungen etc.). Gleichzeitig kann durch das Zusammenwirken die Sensitivität gegenüber dem weiteren Wirkstoff, insbesondere im Fall von Kortikosteroiden (wie z. B. Dexamethason), signifikant gesteigert werden. Diese Wirkungssteigerung lässt sich wiederum möglicherweise - ohne sich auf eine spezielle Theorie festlegen zu wollen - vermutlich auf das von der Anmelderin überraschend aufgefundene steroidartige Wirkungspotential des systemisch eingesetzten 1,8-Cineols erklären, insbesondere im Hinblick auf die Hemmung von Entzündungsmediatoren.

Wie zuvor ausgeführt, kann es im Rahmen der vorliegenden Erfindung vorgesehen sein, dass das 1,8-Cineol als Co-Therapeutikum und/oder als Co-Medikation im Rahmen einer COVID-19-Basistherapie eingesetzt wird und/oder dass das 1,8-Cineol als oder in Kombinationstherapie zu einer COVID-19-Basistherapie und/oder bestehenden COVID-19-Therapie eingesetzt wird. Wie zuvor dargelegt, kann das erfindungsgemäß eingesetzte 1,8-Cineol zu einer signifikanten, insbesondere synergistischen Wirksteigerung des oder der Basistherapeutika, wie insbesondere Kortikosteroide (z. B. Dexamethason), führen.

Gemäß einer weiteren besonderen Ausführungsform der vorliegenden Erfindung kann es vorgesehen sein, dass das 1,8-Cineol zur prophylaktischen und/oder therapeutischen Behandlung, insbesondere zur Suppression oder Abschwächung, eines im Rahmen einer COVID-19-Infektion auftretenden Hyperinflammationssyndroms eingesetzt wird, gegebenenfalls in Co-Medikation mit einem vorzugsweise systemisch applizierten Kortikosteroid, insbesondere Dexamethason.

Weiterhin kann es gemäß einer besonderen Ausführungsform der vorliegenden Erfindung vorgesehen sein, dass das 1,8-Cineol supprimierende oder abschwächende Wirkung in Bezug auf ein im Rahmen einer COVID-19-Infektion auftretendes Hyperinflammationssyndrom ausübt, gegebenenfalls in Co-Medikation mit einem vorzugsweise systemisch applizierten Kortikosteroid, insbesondere Dexamethason.

Weiterhin kann es im Rahmen der vorliegenden Erfindung gemäß einer wiederum weiteren besonderen Ausführungsform vorgesehen sein, dass das 1,8-Cineol zur prophylaktischen und/oder therapeutischen Behandlung, insbesondere zur Suppression oder Abschwächung, einer im Rahmen einer COVID-19-Infektion auftretenden Hyperzytokinämie (Zytokinsturm), insbesondere eines Zytokin-Freisetzungssyndroms (CRS), eingesetzt wird, gegebenenfalls in Co-Medikation mit einem vorzugsweise systemisch applizierten Kortikosteroid, insbesondere Dexamethason.

Gemäß einer wiederum weiteren besonderen Ausführungsform der vorliegenden Erfindung kann es erfindungsgemäß vorgesehen sein, dass das 1,8-Cineol supprimierende oder abschwächende Wirkung in Bezug auf eine im Rahmen einer COVID-19-Infektion auftretende Hyperzytokinämie (Zytokinsturm), insbesondere in Bezug auf ein Zytokin-Freisetzungssyndrom (CRS), ausübt, gegebenenfalls in Co-Medikation mit einem vorzugsweise systemisch applizierten Kortikosteroid, insbesondere Dexamethason.

Wie vorstehend bereits ausgeführt, kann es im Rahmen der vorliegenden Erfindung insbesondere vorgesehen sein, dass das 1,8-Cineol zur Suppression oder Abschwächung einer im Rahmen einer COVID-19-Infektion auftretenden Entzündungsmediation, insbesondere einer im Rahmen einer COVID-19-Infektion auftretenden Hyperzytokinämie (Zytokinsturm), eingesetzt wird.

Ebenfalls kann es im Rahmen der vorliegenden Erfindung insbesondere vorgesehen sein, dass 1,8-Cineol supprimierende oder abschwächende Wirkung in Bezug auf eine im Rahmen einer COVID-19-Infektion auftretende Entzündungsmediation, insbesondere in Bezug auf eine im Rahmen einer COVID-19-Infektion auftretende Hyperzytokinämie (Zytokinsturm), ausübt.

Weiterhin kann es gemäß einer weiteren besonderen Ausführungsform der vorliegenden Erfindung vorgesehen sein, dass das 1,8-Cineol zur Suppression der Replikation von Corona-Viren, insbesondere zur Suppression der in Thrombocyten stattfindenden Replikation von Corona-Viren im Rahmen einer COVID-19-Infektion eingesetzt wird.

Insbesondere kann es im Rahmen der vorliegenden Erfindung vorgesehen sein, dass das 1,8-Cineol supprimierende Wirkung in Bezug auf eine Replikation von Corona-Viren, insbesondere in Bezug auf eine in Thrombocyten stattfindende Replikation von Corona-Viren im Rahmen einer COVID-19-Infektion ausübt. Dieser Aspekt wurde bereits eingangs erläutert und dargelegt.

Ferner kann es im Rahmen der vorliegenden Erfindung gemäß einer wiederum weiteren besonderen Ausführungsform vorgesehen sein, dass das 1,8-Cineol zur prophylaktischen und/oder therapeutischen Behandlung von bei durch Corona-Viren hervorgerufenen Viruserkrankungen, nämlich COVID-19, auftretenden systemischen Organmitbeteiligungen (Organerkrankungen), insbesondere bronchopulmonalen, kardialen und/oder renalen Organmitbeteiligungen (Organerkrankungen), und/oder von bei durch Corona-Viren hervorgerufenen Viruserkrankungen, nämlich COVID-19, auftretenden neurologischen Symptomen, Komplikationen oder Manifestationen (z. B. Verlust bzw. Störungen des Geruchs- und/oder Geschmackssinns, Nervenschädigungen, Kopf- und Muskelschmerzen, Muskelentzündungen etc.) eingesetzt wird. Wie eingangs geschildert, ist die antiinflammatorische, insbesondere die Zytokinproduktion hemmende Wirkung des 1,8-Cineols nicht organspezifisch bzw. nicht lokalspezifisch, so dass sich das 1,8-Cineol für diese spezielle Anwendung bzw. Verwendung in besonderem Maße eignet.

Wie zuvor ebenfalls dargelegt, kann es im Rahmen der vorliegenden Erfindung gleichermaßen vorgesehen sein, dass das 1,8-Cineol als Induktor der NO-Produktion und/oder zur Behebung oder Linderung von bei durch Corona-Viren hervorgerufenen Viruserkrankungen, nämlich COVID-19, auftretenden NO-Defizitzuständen (NO-Mangelsituationen) im Körper eines erkrankten Patienten eingesetzt wird und/oder fungiert. Auf diese Weise wird der Viruserkrankung COVID-19 kausal entgegengewirkt.

Schließlich kann es gemäß einer besonderen Ausführungsform der vorliegenden Erfindung - wie ebenfalls vorstehend erläutert und geschildert - gleichermaßen vorgesehen sein, dass das 1,8-Cineol, zur Behebung oder Linderung von bei durch Corona-Viren hervorgerufenen Viruserkrankungen, nämlich COVID-19, auftretenden oxidativen und/oder inflammatorischen Zuständen im Körper eines erkrankten Patienten eingesetzt wird und/oder fungiert. Auch auf diese Weise wird eine antivirale Therapie der Viruserkrankung bewirkt bzw. realisiert.

Die vorliegende Erfindung, sowohl gemäß dem ersten Aspekt der vorliegenden Erfindung als auch gemäß allen übrigen Aspekten der vorliegenden Erfindung, ist somit mit einer Vielzahl von Vorteilen und Besonderheiten verbunden, welche das erfindungsgemäße Therapiekonzept einzigartig und besonders, insbesondere hocheffizient, machen.

Ebenfalls Gegenstand der vorliegenden Erfindung - gemäß einem **zweiten** Aspekt der vorliegenden Erfindung - ist ein Arzneimittel oder Medikament, insbesondere ein Antivirusmittel, zur Verwendung bei der prophylaktischen und/oder therapeutischen Behandlung von COVID-19, wobei das Arzneimittel oder Medikament, insbesondere das Antivirusmittel, 1,8-Cineol zusammen mit mindestens einem pharmazeutisch verträglichen und/oder physiologisch unbedenklichen Exzipienten (Träger) enthält, wobei das 1,8-Cineol als magensaftresistente, aber dünndarmlösliche systemische Darreichungsform appliziert wird, wobei das Arzneimittel oder Medikament 1,8-Cineol als Reinstoff mit einer Reinheit von mindestens 95 Gew.-%, bezogen auf das 1,8-Cineol, enthält und wobei das Arzneimittel oder Medikament das 1,8-Cineol frei von anderen Terpenen enthält.

Auch im Rahmen dieses Erfindungsaspekt ist es also vorgesehen, dass der Wirkstoff bzw. das 1,8-Cineol bzw. das Arzneimittel oder Medikament, insbesondere Antivirusmittel, systemisch appliziert wird.

Weiterhin kann es auch im Rahmen dieses Erfindungsaspekt erforderlichenfalls vorgesehen sein, dass der Wirkstoff bzw. das 1,8-Cineol bzw. das Arzneimittel oder Medikament, insbesondere Antivirusmittel, zusammen mit mindestens einem weiteren Wirkstoff angewendet oder appliziert wird. Der weitere Wirkstoff kann insbesondere ausgewählt sein aus (i) entzündungshemmenden Wirkstoffen, insbesondere Kortikosteroiden, (ii) blutverdünnenden oder blutgerinnungshemmenden Wirkstoffen, (iiii) antiviral wirkenden Wirkstoffen, insbesondere synthetischen antiviralen Wirkstoffen, wie Remdesivir, oder proteinbasierten antiviralen Wirkstoffen, wie insbesondere monoklonalen Antikörpern, Immunglobulinen und Interferonen; (iv) kardiovaskulären Wirkstoffen, insbesondere Blutdrucksenkern und ACE-Hemmern; sowie deren Kombinationen. Der weitere Wirkstoff wird insbesondere gleichermaßen systemisch angewendet bzw. appliziert.

Erfindungsgemäß bevorzugt ist es, wenn das erfindungsgemäße Arzneimittel oder Medikament, insbesondere Antivirusmittel, das 1,8-Cineol als alleinigen Wirkstoff, insbesondere als alleinigen pharmazeutischen Wirkstoff, enthält.

Gemäß diesem Erfindungsaspekt enthält das erfindungsgemäße Arzneimittel oder Medikament das 1,8-Cineol als Reinstoff mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das 1,8-Cineol und/oder vorzugsweise frei von anderen Terpenen.

Gemäß diesem Erfindungsaspekt ist es weiter vorgesehen, dass das erfindungsgemäße Arzneimittel oder Medikament das 1,8-Cineol frei von anderen Terpenen enthält und/oder dass das erfindungsgemäße Arzneimittel oder Medikament kein weiteres Terpen enthält und/oder dass das erfindungsgemäße Arzneimittel oder Medikament frei von anderen Terpenen als 1,8-Cineol ist.

Gemäß einer wiederum weiteren besonderen Ausführungsform gemäß diesem Erfindungsaspekt kann es vorgesehen sein, dass das erfindungsgemäße Arzneimittel oder Medikament das 1,8-Cineol in wirksamen, insbesondere pharmazeutisch und/oder therapeutisch wirksamen, Mengen enthält.

Insbesondere kann es dabei vorgesehen sein, dass das erfindungsgemäße Arzneimittel oder Medikament das 1,8-Cineol bezogen auf das Arzneimittel oder Medikament, in relativen Mengen im Bereich von 0,0001 bis 80 Gew.-%, insbesondere 0,001 bis 75 Gew.-%, vorzugsweise 0,005 bis 70 Gew.-%, bevorzugt 0,01 bis 60 Gew.-%, besonders bevorzugt 0,05 bis 55 Gew.-%, ganz besonders bevorzugt 0,1 bis 50 Gew.-%, enthält.

Gemäß einer wiederum weiteren besonderen Ausführungsform gemäß diesem Erfindungsaspekt kann es vorgesehen sein, dass der mindestens eine pharmazeutisch verträgliche und/oder physiologisch unbedenkliche Exzipient (Träger) mit 1,8-Cineol mischbar und/oder hierin löslich ist. Vorzugsweise kann dabei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegen. Ebenfalls bevorzugt kann dabei der mindestens eine pharmazeutisch verträgliche und/oder physiologisch unbedenkliche Exzipient (Träger) ausgewählt sein aus der Gruppe von Fettsäureestern, bevorzugt Triglyceriden von Fettsäuren, besonders bevorzugt mittelkettigen Triglyceriden (*Medium Chain Triglycerides* bzw. MCT), ganz besonders bevorzugt Triglyceriden von C₆-C₁₂-Fettsäuren.

Für weitergehende Einzelheiten zu diesem Erfindungsaspekt kann Bezug genommen werden auf die vorstehenden Ausführungen zu den zuvor beschriebenen Erfindungsaspekten, wobei diese Ausführungen geleichermaßen für den vorliegenden Erfindungsaspekt gelten.

Wiederum weiterer Gegenstand der vorliegenden Erfindung - gemäß einem **drit- ten** Aspekt der vorliegenden Erfindung - ist eine Zusammensetzung, insbesondere pharmazeutische Zusammensetzung, zur Verwendung bei der prophylaktischen und/oder therapeutischen Behandlung von COVID-19, wobei die Zusammensetzung 1,8-Cineol, insbesondere zusammen mit mindestens einem pharmazeutisch verträglichen und/oder physiologisch unbedenklichen Exzipienten (Träger), enthält wobei das 1,8-Cineol als magensaftresistente, aber dünndarmlösliche systemische Darreichungsform appliziert wird, wobei das Arzneimittel oder Medikament 1,8-Cineol als Reinstoff mit einer Reinheit von mindestens 95 Gew.-%, bezogen auf das 1,8-Cineol, enthält und wobei das Arzneimittel oder Medikament das 1,8-Cineol frei von anderen Terpenen enthält.

Auch im Rahmen dieses Erfindungsaspekt ist es also vorgesehen, dass der Wirkstoff bzw. das 1,8-Cineol bzw. die Zusammensetzung, insbesondere pharmazeutische Zusammensetzung, systemisch appliziert wird.

Weiterhin kann es auch im Rahmen dieses Erfindungsaspekt erforderlichenfalls vorgesehen sein, dass der Wirkstoff bzw. das 1,8-Cineol, bzw. die Zusammensetzung, insbesondere pharmazeutische Zusammensetzung, zusammen mit mindestens einem weiteren Wirkstoff angewendet oder appliziert wird. Der weitere Wirkstoff kann insbesondere ausgewählt sein aus (i) entzündungshemmenden Wirkstoffen, insbesondere Kortikosteroiden, (ii) blutverdünnenden oder blutgerinnungshemmenden Wirkstoffen, (iiii) antiviral wirkenden Wirkstoffen, insbesondere synthetischen antiviralen Wirkstoffen, wie Remdesivir, oder proteinbasierten antiviralen Wirkstoffen, wie insbesondere monoklonalen Antikörpern, Immunglobulinen und Interferonen; (iv) kardiovaskulären Wirkstoffen, insbesondere Blutdrucksenkern und ACE-Hemmern; sowie deren Kombinationen. Der weitere Wirkstoff wird insbesondere gleichermaßen systemisch angewendet bzw. appliziert.

Erfindungsgemäß bevorzugt ist es, wenn die erfindungsgemäße Zusammensetzung, insbesondere pharmazeutische Zusammensetzung, das 1,8-Cineol, als alleinigen Wirkstoff, insbesondere als alleinigen pharmazeutischen Wirkstoff, enthält.

Gemäß diesem Erfindungsaspekt enthält die erfindungsgemäße Zusammensetzung, insbesondere pharmazeutische Zusammensetzung, das 1,8-Cineol als Reinstoff mit einer Reinheit von mindestens 95 Gew.-%, insbesondere mindestens 96 Gew.-%, bevorzugt mindestens 97 Gew.-%, besonders bevorzugt mindestens 98 Gew.-%, ganz besonders bevorzugt mindestens 99 Gew.-%, noch mehr bevorzugt mindestens 99,5 %, bezogen auf das 1,8-Cineol, und frei von anderen Terpenen.

Gemäß diesem Erfindungsaspekt ist es also vorgesehen, dass die erfindungsgemäße Zusammensetzung, insbesondere pharmazeutische Zusammensetzung, das 1,8-Cineol frei von anderen Terpenen enthält und/oder dass die erfindungsgemäße Zusammensetzung, insbesondere pharmazeutische Zusammensetzung, kein weiteres Terpen enthält und/oder dass die erfindungsgemäße Zusammensetzung, insbesondere pharmazeutische Zusammensetzung, frei von anderen Terpenen als 1,8-Cineol ist.

Gemäß einer wiederum weiteren besonderen Ausführungsform gemäß diesem Erfindungsaspekt kann es vorgesehen sein, dass die erfindungsgemäße Zusammensetzung, insbesondere pharmazeutische Zusammensetzung, das 1,8-Cineol in wirksamen, insbesondere pharmazeutisch und/oder therapeutisch wirksamen, Mengen enthält.

Insbesondere kann es dabei vorgesehen sein, dass die erfindungsgemäße Zusammensetzung, insbesondere pharmazeutische Zusammensetzung, das 1,8-Cineol bezogen auf die Zusammensetzung, in relativen Mengen im Bereich von 0,0001 bis 80 Gew.-%, insbesondere 0,001 bis 75 Gew.-%, vorzugsweise 0,005 bis 70 Gew.-%, bevorzugt 0,01 bis 60 Gew.-%, besonders bevorzugt 0,05 bis 55 Gew.-%, ganz besonders bevorzugt 0,1 bis 50 Gew.-%, enthält.

Gemäß einer wiederum weiteren besonderen Ausführungsform gemäß diesem Erfindungsaspekt kann es vorgesehen sein, dass der mindestens eine pharmazeutisch verträgliche und/oder physiologisch unbedenkliche Exzipient (Träger) mit 1,8-Cineol mischbar und/oder hierin löslich ist. Vorzugsweise kann dabei der mindestens eine pharmazeutisch verträgliche und/oder physiologisch unbedenkliche Exzipient (Träger) bei 20 °C und bei Atmosphärendruck im flüssigen oder festen, vorzugsweise flüssigen, Aggregatzustand vorliegen. Ebenfalls bevorzugt kann dabei der mindestens eine pharmazeutisch verträgliche und/oder physiologisch unbedenkliche Exzipient (Träger) ausgewählt sein aus der Gruppe von Fettsäureestern, bevorzugt Triglyceriden von Fettsäuren, besonders bevorzugt mittelkettigen Triglyceriden (*Medium Chain Triglycerides* bzw. MCT), ganz besonders bevorzugt Triglyceriden von C₆-C₁₂-Fettsäuren.

Für weitergehende Einzelheiten zu diesem Erfindungsaspekt kann Bezug genommen werden auf die vorstehenden Ausführungen zu den zuvor beschriebenen Erfindungsaspekten, wobei diese Ausführungen geleichermaßen für den vorliegenden Erfindungsaspekt gelten.

Schließlich ist Gegenstand der vorliegenden Erfindung - gemäß einem **vierten** Aspekt der vorliegenden Erfindung - gleichermaßen eine pharmazeutische Kombination zur Verwendung bei der prophylaktischen und/oder therapeutischen Behandlung von COVID-19,
wobei die pharmazeutische Kombination mindestens die nachfolgenden Komponenten (A) und (B) umfasst:
(A) 1,8-Cineol; sowie
(B) mindestens einen weiteren Wirkstoff, ausgewählt aus (i) entzündungshemmenden Wirkstoffen, insbesondere Kortikosteroiden, (ii) blutverdünnenden oder blutgerinnungshemmenden Wirkstoffen, (iiii) antiviral wirkenden Wirkstoffen, insbesondere synthetischen antiviralen Wirkstoffen, wie Remdesivir, oder proteinbasierten antiviralen Wirkstoffen, wie insbesondere monoklonalen Antikörpern, Immunglobulinen und Interferonen; (iv) kardiovaskulären Wirkstoffen, insbesondere Blutdrucksenkern und ACE-Hemmern; sowie deren Kombinationen
wobei das 1,8-Cineol als magensaftresistente, aber dünndarmlösliche systemische Darreichungsform appliziert wird, wobei das Arzneimittel oder Medikament 1,8-Cineol als Reinstoff mit einer Reinheit von mindestens 95 Gew.-%, bezogen auf das 1,8-Cineol, enthält und wobei das Arzneimittel oder Medikament das 1,8-Cineol frei von anderen Terpenen enthält.

Auch im Rahmen dieses Erfindungsaspekt ist es vorgesehen, dass der Wirkstoff der Komponente (A) bzw. das 1,8-Cineol systemisch appliziert wird.

Auch der weitere Wirkstoff der Komponente (B) kann insbesondere gleichermaßen systemisch angewendet bzw. appliziert werden.

Gemäß einer besonderen Ausführungsform dieses Erfindungsaspekts kann es insbesondere vorgesehen sein, dass die Komponenten (A) und (B) getrennt voneinander, insbesondere räumlich getrennt voneinander, aber funktional zusammenhängend und/oder funktional einander zugeordnet vorliegen und/oder verabreicht werden.

Weiterhin kann es gemäß einer wiederum besonderen Ausführungsform dieses Erfindungsaspekts insbesondere vorgesehen sein, dass die pharmazeutische Kombination in Form eines Kit (d. h. Kit-of-parts), insbesondere als Kit der Komponenten (A) und (B), vorliegt und/oder dass die Komponenten (A) und (B) als Kit (d. h. Kit-of-parts) vorliegen und/oder hergerichtet sind und/oder verabreicht werden.

Als Kit bzw. Kit-of-parts wird im Rahmen der vorliegenden Erfindung insbesondere eine Einheit bzw. Anordnung bzw. Zusammenstellung bzw. Kombination der beiden Komponenten (A) und (B) verstanden, bei welcher die beiden Komponenten (A) und (B) zwar getrennt und/oder separat, insbesondere räumlich getrennt und/oder räumlich separiert, vorliegen, aber als funktional zusammenhängende bzw. als funktional einander zugeordnete Komponenten vorgesehen sind bzw. verabreicht werden.

Für weitergehende Einzelheiten zu diesem Erfindungsaspekt kann Bezug genommen werden auf die vorstehenden Ausführungen zu den zuvor beschriebenen Erfindungsaspekten, wobei diese Ausführungen geleichermaßen für den vorliegenden Erfindungsaspekt gelten.

Die folgenden Ausführungsbeispiele dienen lediglich der Veranschaulichung der vorliegenden Erfindung, jedoch ohne die vorliegende Erfindung hierauf zu beschränken.

### AUSFÜHRUNGSBEISPIELE:

### Beispiel 1: Studien und Beobachtungen in Bezug auf Thrombocyten und Entzündungsmediation in Verbindung mit einer Cineoltherapie

Wie eingangs bereits erläutert und wie von Anmelderin in diesem Zusammenhang überraschend gefunden, können Thrombozyten (Blutplättchen), welche neben der Blutgerinnung auch als immunregulatorische Entzündungszellen fungieren, Coronaviren beherbergen und somit durch Coronaviren ausgelöste Virusinfektionen, insbesondere COVID-19, triggern, wobei der Wirkstoff 1,8-Cineol insbesondere einem im Rahmen der Coronavirusinfektion auftretetenden Hyperinflammationssyndrom und vor allem einer Hyperzytokinämie (Zytokinsturm) entgegenwirken kann und die Coronavirusinfektion einzudämmen imstande ist. Bei bisherigen COVID-19-Infektionsverläufen ist bekannt, dass insbesondere zwei Zustände der Entzündung eintreten können, nämlich derjenige, welcher zu einer Hyperinflammation mit potentiell kritischem Verlauf führt, oder derjenige, welcher zu einer viralen Clearance führt. Vor allem sind Thrombozyten aus der Blutgerinnung und der Wahrung der vaskulären Integrität bekannt; aber jüngere Studien zeigen auch, dass Thrombozyten wichtige aktive Regulatoren des Immunsystems sind und insbesondere dem angeborenen Immunsystem zuzurechnen sind. In diesem Zusammenhang konnten die Bedeutung der Thrombozyten an entzündlich bedingten Erkrankungen nachgewiesen und auf diese Weise Einblicke in die Pathomechanismen erhalten werden.

Thrombozyten sind im Zusammenhang von viralen Infektionen insbesondere in der Lage, massive Entzündungsreaktionen (insbesondere eine Hyperzytokinämie) zu induzieren und aufrechtzuerhalten sowie Antigene zu präsentieren und Immunreaktionen in viralen Erkrankungen zu starten, aber auch RNA aufzunehmen und diese horizontal an andere Zellen weiterzugeben und darüber hinaus RNA-Viren aufzunehmen und ihnen einen Replikationsort zu bieten. Des Weiteren sind Thrombozyten imstande, sogenannte plättchen- bzw. thrombozytenderivierte Mikrovesikel (*plateletderived microparticles* bzw. *PMP*) zu produzieren und durch PMP-Immunzellen zu regulieren. Aufgrund von klinischen Beobachtungen bei COVID-19-Infektionen können Thrombocyten als zentrale Zielzellen einer COVID-19-Infektion angesehen bzw. bewertet werden: So kann ein verändertes Thrombozyten/Lymphozyten-Verhältnis (sogenanntes *platelet-to-lymphocyte ratio* bzw. *PTR*) als inflammatorischer Marker und prognostischer Faktor in Bezug auf den Verlauf von COVID-19-Infektionen herangezogen werden. Auch ein verändertes Koagulationsverhalten lässt den Hinweis zu, dass PTL-Inhibition mit einem milderen Verlauf bei COVID-19-Infektionen verbunden ist. Eine Thrombozytopenie dagegen kann als Marker für einen schweren COVID-19-Verlauf gewertet werden. COVID-19-Infektionen sind im Allgemeinen durch eine Vielzahl proinflammatorische Effektor-Zytokine gekennzeichnet, welche aus Thrombozyten freigesetzt werden, wie z. B. TNF, IL-1β, IL-6, IL-8, G-CSF und GM-CSF.

Im Rahmen der vorliegenden Versuche kann durch die Anmelderin ein grundlegendes klinisches und immunologisches Verständnis in Bezug auf die Alteration von Thrombocyten durch 1,8-Cineol im Kontext von COVID-19-Infektionen unter Cineolbehandlung gewonnen werden. In diesem Zusammenhang kann seitens der Anmelderin gezeigt werden, dass Cineol, nämlich 1,8-Cineol, effizient in der Behandlung von durch Corona-Viren hervorgerufenen Viruserkrankungen, nämlich COVID-19, eingesetzt werden kann.

Im Rahmen von in-vitro-Studien mit aus gesunden Probanden gewonnenen Thrombocyten, welche zunächst mit 1,8-Cineol inkubiert und dann mit Corona-Viren infiziert werden, kann gezeigt werden, dass sich ein Infektionsausbruch, insbesondere eine Hyperzytokinämie, präventiv verhindern bzw. vermindern lässt.

In weiteren *in*-*vitro*-Studien mit aus an COVID-19 erkrankten Patienten gewonnenen Thrombocyten, welche mit 1,8-Cineol inkubiert werden, lassen sich diese Ergebnisse bestätigen, insbesondere dahingehend, dass sich der Infektionsverlauf, insbesondere eine Hyperzytokinämie, verhindern bzw. abschwächen lässt. Diese antivirale Wirkung von 1,8-Cineol bei COVID-19-Infektionen ist vollkommen unerwartet.

In diesem Zusammenhang ist es bekannt, dass Thrombozyten an Lungenepithelien adhärieren und auf diese Weise eine Infektion der Epithelien mit COVID-19 ermöglichen bzw. exazerbieren. Die Adhäsion an Epithelien ist wiederum zytokinabhängig und kann folglich durch Cineol, nämlich 1,8-Cineol, inhibiert bzw. verringert oder abgeschwächt werden. Damit wird auch eine Infektion der Epithelien und letztlich des Lungengewebes abgemildert bzw. verhindert.

### Analyse der Veränderung von Thrombocyten COVID-19-positiver Patienten

Es wird die Veränderung der Thrombocyten in den drei Stadien, d. h. Anschoppung, Vollform der Infektion und post-Infektion, untersucht, insbesondere speziell in dem Kontext der entzündungsmediierenden Aktivierung. Im Einzelnen werden untersucht: die RNA-Textur, das Proteom, das Metabolom, die Existenz von *platelet-derived microparticles.*

Gesunde Thrombocyten werden mit COVID-19 inkubiert und funktionell (d. h. in Bezug auf Zytokine) untersucht. Anschließend kann das Virus sowohl in seiner Replikation als auch in der Produktion der in Thrombocyten ausgelösten hyperinflammatorischen Zytokine, welche zum sogenannten Zytokinsturm führen, durch Inkubation mit 1,8-Cineol gehindert werden.

Im Einzelnen werden in diesem Zusammenhang:
- initial gesunde Thrombocyten mit 1,8-Cineol inkubiert, dann mit dem Virus infiziert, um präventiv zu zeigen, dass sich die Infektion verhindern bzw. vermindern lässt; und
- COVID-19-infizierte Thrombocyten mit 1,8-Cineol inkubiert, um zu zeigen, dass sich ein Zytokinsturm verhindern lässt.

Im Rahmen der vorliegenden Versuche kann somit durch die Anmelderin überraschend ein grundlegendes klinisches und immunologisches Verständnis in Bezug auf die Alteration von Thrombocyten im Kontext von COVID-19-Infektionen unter Cineolbehandlung gewonnen werden. In diesem Zusammenhang kann seitens der Anmelderin gezeigt werden, dass Cineol, nämlich 1,8-Cineol, effizient in der Behandlung von durch Corona-Viren hervorgerufenen Viruserkrankungen, nämlich COVID-19, eingesetzt werden kann.

### Beispiel 2: Studien und Beobachtungen in Bezug auf die Hemmung der Produktion von Entzündungsmediatoren, insbesondere Zytokinen, durch Cineol

Wie eingangs ebenfalls bereits erläutert und wie von Anmelderin in diesem Zusammenhang gleichermaßen überraschend gefunden, bewirkt der Wirkstoff 1,8-Cineol gleichermaßen eine Hemmung in Bezug auf die Produktion von Entzündungsmediatoren, insbesondere Zytokinen, und kann somit im Rahmen einer COVID-19-Therapie eine Hyperzytokinämie (Zytokinsturm) entgegenwirken.

Cineol, nämlich 1,8-Cineol, insbesondere in Reinform, bewirkt in diesem Zusammenhang eine signifikante Hemmung der Mediatorproduktion; hierdurch wird somit ein entzündungshemmender Effekt bewirkt. Dieser Effekt wird bei systemischer Applikation von Cineol, nämlich 1,8-Cineol, im gesamten Körper und vor allem in den gesamten Atemwegen, insbesondere in der gesamten Lunge, d. h. auch bis in die Peripherie und die Alveolen, erreicht. Bei ätherischen Mischölen ist dieser Effekt signifikant schlechter ausgeprägt.

Cineol, nämlich 1,8-Cineol, insbesondere in Reinform, bewirkt in diesem Zusammenhang eine signifikante Hemmung der Mediatorproduktion: Selbst niedrige therapeutische Konzentrationen von 1,8-Cineol können bereits die Produktion von IL-1beta (Interleukin-1beta) im Vergleich zu subtherapeutischen Konzentrationen von Kortikosteroiden (z. B. insbesondere Dexamethason), für welche keine signifikante Hemmung nachgewiesen werden, dennoch signifikant hemmen, d. h. durch Kombination von 1,8-Cineol mit den betreffenden Kortikosteroiden (z. B. insbesondere Dexamethason) kann eine signifikante Hemmung sogar für subtherapeutische, aber selbstverständlich auch für therapeutische Dosen bzw. Konzentrationen von Kortikosteroiden (z. B. insbesondere Dexamethason) mit einer resultierenden Intensivierung der Wirkung der Kortikosteroide nachgewiesen werden.

Die nachfolgende Tabelle 1 zeigt die hemmende Aktivität von 1,8-Cineol als Reinstoff (10⁻⁶ mol/l) einerseits und Dexamethason andererseits sowie deren Kombinationen in Bezug auf die LPS-stimulierte Produktion von IL-1beta in humanen Monozyten in vitro. Die monozytäre IL-1beta-Produktion (n = 14, 4 Experimente) in Monozyten wird durch Cineol (10⁻⁶ mol/l) im Vergleich zur Kontrolle signifikant gehemmt. Mischöl (Eukalyptusöl) mit nur ca. 60 % 1,8-Cineol in derselben Konzentration (10⁻⁶ mol/l) hemmt die IL-1beta-Produktion signifikant schwächer als 1,8-Cineol als Reinstoff. Dexamethason in subtherapeutischen Dosen (10⁻⁹ mol/l) bewirkt keine signifikante Hemmung. Cineol und Dexamethason in Kombination dagegen hemmen synergistisch die IL-1beta-Produktion, d. h. stärker als Dexamethason allein (und zwar auch schon bei subtherapeutischen Konzentrationen von Dexamethason von nur 10⁻⁹ mol/l). Im Vergleich zu Cineol (10⁻⁶ mol/l) allein wird die IL-1beta-Produktion durch Zusatz von Dexamethason synergistisch und signifikant stärker gehemmt (p < 0,05).

Die in Tabelle 1 dargestellten Ergebnisse aus humanen Monozytenkulturen zeigen, dass die LPS-stimulierte Produktion von IL-1beta durch 1,8-Cineol signifikant gehemmt wird und dass die LPS-stimulierte Produktion von IL-1beta durch eine Kombination von 1,8-Cineol und Dexamethason signifikant stärker gehemmt wird als durch Dexamethason allein oder auch als durch 1,8-Cineol allein (siehe nachfolgende Tabelle 1).

In diesem Zusammenhang zeigen die Versuche eine hemmende Wirkung in Bezug auf die Produktion von IL-1beta durch das Monoterpen 1,8-Cineol einerseits und durch das Kortikosteroid Dexamethason andererseits sowie deren Kombination in LPS-stimulierten humanen Monozyten in vitro. Die Zugabe bereits geringer Mengen von 1,8-Cineol bewirkt eine signifikante Steigerung der Wirkung des Kortikosteroids, einhergehend mit einer gesteigerten Hemmung von IL-1beta: Monozyten (n = 14, 4 Experimente) von gesunden Probanden (10⁵ / ml) werden über 20 Stunden mit 1,8-Cineol (10⁻⁶ mol/l = 0,015 g/ml) und dem Kortikosteroid Dexamethason (10⁻¹² mol/l bzw. 10⁻⁹ mol/l) sowie deren Kombination in Gegenwart von LPS (10 mg/ml) inkubiert. In Zellkulturüberständen wird die Produktion von IL-1beta durch ELISA (Fa. Cayman Chemical, Ann Arbor, Michigan, USA) bestimmt. 1,8-Cineol allein und therapeutisch relevante Konzentrationen von Dexamethason allein (10⁻⁹ mol/l) hemmen jeweils signifikant die Produktion von IL-1beta. Dagegen wird die LPS-stimulierte Produktion durch eine Kombination von 1,8-Cineol (10⁻⁶ mol/l) plus Dexamethason signifikant stärker gehemmt als durch Dexamethason allein. 1,8-Cineol intensiviert insbesondere die Wirkung bereits subtherapeutischer Konzentrationen von Dexamethason.

**Tabelle 1: Hemmende Aktivität von 1,8-Cineol (Cineol), Dexamethason (Dex.) und Kombinationen von 1,8-Cineol und Dexamethason in Bezug auf die LPS-stimulierte Produktion von IL-1beta in humanen Monozyten**

| **Konzentration mol/l** | **IL-1beta pg / 5 × 10⁻⁴ Zellen** | **Effekt vs. Kontrolle** | |
|---|---|---|---|
| | | **Hemmung (%)** | **p-Wert** |
| **Kontrolle** | **5252 ± 1017** | **0 ± 19** | **-** |
| **Mischöl 10⁻¹ (Eukalyptusöl)** | **4732 ± 473** | **9,9 ± 10** | **0,1901** |
| **Cineol 10⁻⁶** | **3548 ± 600** | **32,4 ± 17** | **0,0100** |
| **Dex. 10⁻¹²** | **5245 ± 1027** | **0,1 ± 19** | **0,9634** |
| **Dex. 10⁻⁹** | **2655 ± 546** | **49,5 ± 20** | **0,0449** |
| **Cineol 10⁻⁶ + Dex. 10⁻¹²** | **3965 ± 641** | **24,6 ± 16** | **0,1904** |
| **Cineol 10⁻⁶ + Dex. 10⁻⁹** | **348 ± 55** | **93,5 ± 16** | **0,0049** |

Die Studien der Anmelderin zeigen somit insgesamt, dass der Wirkstoff Cineol, nämlich 1,8-Cineol, vor allem in Form der Reinsubstanz, imstande ist, bei der prophylaktischen und/oder therapeutischen Behandlung von durch Corona-Viren hervorgerufenen Viruserkrankungen, nämlich COVID-19, den betreffenden Infektionsverlauf abzuschwächen bzw. abzuwehren, insbesondere eine Hyperzytokinämie zu verhindern bzw. abzuschwächen. Diese antivirale Wirkung von 1,8-Cineol bei COVID-19-Infektionen ist vollkommen unerwartet und lässt sich durch systemische Gabe von Kortikosteroiden (z. B. insbesondere Dexamethason) sogar noch weiterführend steigern, insbesondere in synergistischer Weise.

### Beispiel 3: Studien und Beobachtungen in Bezug auf eine antioxidative und NO-regulative Wirkung von Cineol

Wie eingangs gleichermaßen dargelegt und von der Anmelderin gleichermaßen überraschend gefunden, wirkt 1,8-Cineol auch als Antioxidans und NO-Regulator in Bezug auf bei durch Corona-Viren hervorgerufenen Viruserkrankungen, nämlich COVID-19, auftretende Oxidationsprozesse und NO-Mangelzustände im Körper bzw. in den Organen der betroffenen Patienten.

NO als solches wirkt als antiinflammatorischer Mediator sowie als Hemmstoff von Entzündungsmediatoren und der Thrombozytenaggregation und als Aktivator der ziliären Funktion und mukosalen Clearance und schützt insbesondere vor respiratorischen Infekten und Exazerbationen respiratorischer Infekte. In dieser Hinsicht ist Cineol, nämlich 1,8-Cineol, geeignet, um als Wirkstoff bzw. Therapeutikum die bei Coronavirus-Infektionen vorliegende supprimierte NO-Produktion durch günstigen Abbau von O₂⁻-Radikalen mit Induktion von NO zu normalisieren und den jeweiligen Erfordernissen entsprechend durch Modulation adäquat anzupassen.

Im Rahmen der vorliegenden Erfindung können modulierende antioxidative und antiinflammatorische Wirkungen von 1,8-Cineol zur Kontrolle oxidativer Prozesse und Induktion der Stickoxidproduktion (NO-Produktion) nachgewiesen werden. Auf der Suche nach der zugrundeliegenden Eigenschaft von 1,8-Cineol zur Intensivierung antiinflammatorischer Wirkungen bei der Therapie von COVID-19 wird eine antioxidative Wirkung für 1,8-Cineol durch Hemmung der Produktion von Superoxiden (O₂⁻-Radikale), der Aktivität von Superoxiddismutasen (SOD) und von Wasserstoffperoxid (H₂O₂) gefunden. Hierbei wird eine Hemmung der spontanen und stimulierten Produktion von Superoxiden (O₂⁻-Radikalen) bei therapeutischen Konzentrationen von 1,8-Cineol nachgewiesen, so dass die Bereitstellung des Substrats für die Dismutierung von O₂⁻-Radikalen über eine durch 1,8-Cineol partiell gehemmte Superoxid-Dismutaseaktivität auch bei niedrigeren Konzentrationen und im therapeutischen Bereich die Produktion von O₂⁻-Radikalen und H₂O₂ hemmt. Als wesentliche Ursache für diese antioxidative Wirkung von 1,8-Cineol wird überraschend dessen Eigenschaft als aktiver Induktor der NO-Produktion gefunden, d. h. 1,8-Cineol ist imstande, aktiv die NO-Produktion durch Vermittlung antioxidativer Wirkungen zu induzieren (siehe nachfolgende Tabelle 2).

Die nachfolgende Tabelle 2 zeigt den Effekt von 1,8-Cineol auf die PMA-stimulierte Superoxid-Produktion (O₂⁻-Produktion) (in RPMI-1640) humaner Monozyten in vitro. Die dosisabhängigen Effekte von 1,8-Cineol (4 Experimente, n = 14) auf die O₂⁻-Produktion werden durch Bestimmung von INT-Formazan in Kulturüberständen (RPMI-1640) humaner Monozyten (10⁵ / ml) nach 20 Stunden gemessen. Die O₂⁻-Produktion wird nicht durch PMA (500 mmol/l) stimuliert. Für die statistische Analyse wird der nichtparametrische Mann & Whitney-Test herangezogen (p < 0,05). Hierbei können modulierende Einflüsse von 1,8-Cineol zur Kontrolle oxidativer und somit zellschädigender und proinflammatorischer Einflüsse (wie sie auch bei COVID-19 auftreten) durch Hemmung von O₂⁻-Radikalen und durch eine gegenläufige Stimulation von antiinflammatorischem NO im therapeutischen Bereich von 1,8-Cineol nachgewiesen werden.

**Tabelle 2: Effekt von 1,8-Cineol auf die PMA-stimulierte Superoxidproduktion (O₂⁻-Produktion) in RPMI 1640 humaner Monozyten in vitro**

| **1,8-Cineol g/ml (mol/l)** | **INT-Formazan (nmol/10⁵)** | **Vergleich zur Kontrolle (%)** | **p-Wert** |
|---|---|---|---|
| **spontan** | **4828 ± 251** | **-** | **-** |
| **PMA** | **4203 ± 267** | **-12,9 ± 6** | **0,0990** |
| **0,0015 (10⁻⁸)** | **5364 ± 229** | **+27,6 ± 4** | **0,0083** |
| **1,2 (8 x 10⁻⁶)** | **4099 ± 234** | **-2,5 ± 6** | **0,7721** |
| **1,5 (10⁻⁵)** | **2438 ± 389** | **-42 ± 16** | **0,0024** |
| **3,0 (2 x 10⁻⁵)** | **634 ± 139** | **-84,9 ± 22** | **< 0,0001** |

Die vorstehende Tabelle 2 zeigt somit den konzentrationsabhängigen modulierenden Effekt von 1,8-Cineol auf die O₂⁻- und NO-Produktion in stimulierten humanen Monozyten in vitro: Nach Stimulation (20 Stunden) normaler humaner Monozyten (10⁵ / ml) wird in der Kontrolle (d. h. ohne 1,8-Cineol) die Produktion von NO induziert und von O₂⁻ supprimiert. Niedrige subtherapeutische Konzentrationen von 1,8-Cineol dagegen induzieren leichtgradig die O₂⁻-Produktion, aber hemmen bereits die NO-Produktion. Im therapeutischen Dosisbereich von 1,8-Cineol dagegen wird die O₂⁻-Produktion stark gehemmt, und zwar in Gegenwart stark bzw. siginifikant stimulierender Effekte auf die NO-Produktion.

Diese Ergebnisse sind auch von integraler Bedeutung für das Verständnis in Bezug auf die Therapie von Coronavirus-Infektionen, insbesondere COVID-19, mit 1,8-Cineol: So wird eine erhöhte Produktion von O₂⁻-Radikalen, wie sie bei Coronavirus-Infektionen, insbesondere COVID-19, auftritt, durch Therapie mit 1,8-Cineol dauerhaft gehemmt und außerdem zudem vorteilhaft als Substrat für die Produktion von NO genutzt. NO wiederum wirkt als antiinflammatorischer Mediator sowie als Hemmstoff von Entzündungsmediatoren und der Thrombozytenaggregation sowie als Aktivator der ziliären Funktion und mukosalen Clearance und schützt zusammenfassend vor respiratorischen Infekten und deren Exazerbationen. In dieser Hinsicht ist 1,8-Cineol geeignet, als COVID-19-Therapeutikum die bei COVID-19 supprimierte NO-Produktion durch günstigen Abbau von O₂⁻-Radikalen mit Induktion von NO zu normalisieren und den jeweiligen Erfordernissen entsprechend durch Modulation adäquat anzupassen.

Insgesamt eignet sich somit Cineol, nämlich 1,8-Cineol, auf Basis der überraschend aufgefundenen Erkenntnisse der Anmelderin in effizienter Weise zur Verwendung bei der prophylaktischen und/oder therapeutischen Behandlung von durch Corona-Viren hervorgerufenen Viruserkrankungen, nämlich COVID-19 (z. B. als alleiniges Therapeutikum oder aber in Co-Therapie mit weiteren Wirkstoffen, wie zuvor beschrieben, insbesondere Kortikosteroiden, wie z. B. Dexamethason).

### Beispiel 4: Weitere Studien und Beobachtungen in Bezug auf die Hemmung der durch COVID-19 bzw. SARS-CoV-2 induzierten Monozytenaktivierung durch Cineol

Wie von der Anmelderin gleichermaßen überraschend gefunden, bewirkt 1,8-Cineol gleichermaßen eine Hemmung der durch COVID-19 bzw. SARS-CoV-2 induzierten Monozytenaktivierung.

Als SARS-CoV-2-Pseudovirus wird das Spike-Protein genutzt, da schon beschrieben ist, dass Proteine aus der Virushülle eine Immunantwort hervorrufen. Dies wurde schon bei Thrombozyten, Macrophagen und anderen Zellen beschrieben. Folglich konnte auch mittels einer Docking-Studie gezeigt werden, dass das Spike-Protein an *Toll*-*like*-Rezeptoren (TLR) binden und so einen gravierenden Entzündungsmechanismus in der Zelle auslösen kann.

Die Anmelderin kann im Rahmen der vorliegenden Erfindung nachweisen, dass 1,8-Cineol die SARS-CoV-2-induzierte Monozytenaktivierung hemmt. Da das Virusprotein hauptsächlich die NFkB- und MAPK-Signalwege in Monozyten aktiviert und diese Signalwege in Thrombozyten durch 1,8-Cineol inhibiert werden können, kann im Rahmen der vorliegenden Erfindung insbesondere gezeigt werden, dass 1,8-Cineol auch in Monozyten inflammatorische Signalwege hemmen kann.

Zu diesem Zweck werden Monozyten (unbehandelt = Vergleich oder vorbehandelt mit 1 mM 1,8-Cineol für 1 h) jeweils mit 5 µg / mL S1-Spike-Protein stimuliert. Die Gesamtproteinlevel und die phosphorylierten Proteinlevel der NFkB- und MAPK-Signalwege werden mittels Western-Blot-Analyse bestimmt.

Die Ergebnisse zeigen, dass 1,8-Cineol die S1-Aktivierung signifikant inhibiert. Hauptsächlich können die Phosphorylierungen von IkBa, NFkB, Akt und Erk1/2 inhibiert werden. Da der NFkB-Signalweg grundsätzlich dafür verantwortlich ist, pro-inflammatorische Gene zu exprimieren und folglich pro-inflammatorische Zytokine zu produzieren und zu sekretieren, folgt hieraus unmittelbar auch, dass auch die Zytokinsekretierung unter Cineol vermindert ist.

Weiterführende Experimente der Anmelderin zum inhibitorischen Effekt von 1,8-Cineol bei SARS-CoV-2-aktivierten Monozyten haben diese Ergebnisse bestätigt.

## Patentansprüche

1. 1,8-Cineol zur Verwendung bei der prophylaktischen und/oder therapeutischen Behandlung von COVID-19,
wobei das 1,8-Cineol als magensaftresistente, aber dünndarmlösliche systemische Darreichungsform appliziert wird,
wobei das 1,8-Cineol als Reinstoff vorliegt, wobei das 1,8-Cineol mit einer Reinheit von mindestens 95 Gew.-%, bezogen auf das 1,8-Cineol, vorliegt und wobei das 1,8-Cineol frei von anderen Terpenen ist.

2. 1,8-Cineol zur Verwendung nach Anspruch 1,
wobei das 1,8-Cineol als Kapsel, Dragee, Pille oder Tablette appliziert wird.

3. 1,8-Cineol zur Verwendung nach Anspruch 1 oder 2,
wobei das 1,8-Cineol mit einer Tagesdosis im Bereich von 1 bis 5.000 mg/diem, insbesondere im Bereich von 2 bis 3.000 mg/diem, vorzugsweise im Bereich von 5 bis 2.500 mg/diem, bevorzugt im Bereich von 10 bis 2.000 mg/diem, besonders bevorzugt im Bereich von 50 bis 1.500 mg/diem, verabreicht wird.

4. 1,8-Cineol zur Verwendung nach einem der vorangehenden Ansprüche,
wobei das 1,8-Cineol, zusammen mit mindestens einem physiologisch unbedenklichen Träger vorliegt und/oder verabreicht wird;
insbesondere wobei der physiologisch unbedenkliche Träger mit 1,8-Cineol mischbar und/oder hierin löslich ist; und/oder
insbesondere wobei der physiologisch unbedenkliche Träger bei 20 °C und bei Atmosphärendruck im flüssigen oder festen, vorzugsweise flüssigen, Aggregatzustand vorliegt; und/oder
insbesondere wobei der physiologisch unbedenkliche Träger ausgewählt ist aus der Gruppe von Fettsäureestern, bevorzugt Triglyceriden von Fettsäuren, besonders bevorzugt mittelkettigen Triglyceriden, ganz besonders bevorzugt Triglyceriden von C₆-C₁₂-Fettsäuren.

5. 1,8-Cineol zur Verwendung nach einem der vorangehenden Ansprüche,
wobei das 1,8-Cineol zusammen mit mindestens einem weiteren Wirkstoff angewendet oder appliziert wird;
insbesondere wobei der weitere Wirkstoff ausgewählt ist aus (i) entzündungshemmenden Wirkstoffen, insbesondere Kortikosteroiden, (ii) blutverdünnenden oder blutgerinnungshemmenden Wirkstoffen, (iiii) antiviral wirkenden Wirkstoffen, insbesondere synthetischen antiviralen Wirkstoffen, wie Remdesivir, oder proteinbasierten antiviralen Wirkstoffen, wie insbesondere monoklonalen Antikörpern, Immunglobulinen und Interferonen; (iv) kardiovaskulären Wirkstoffen, insbesondere Blutdrucksenkern und ACE-Hemmern; sowie deren Kombinationen; und/oder
insbesondere wobei der weitere Wirkstoff getrennt, insbesondere räumlich getrennt, von dem 1,8-Cineol aber funktional zusammenhängend hiermit eingesetzt und/oder verabreicht wird, insbesondere in Form eines Kit.

6. 1,8-Cineol zur Verwendung nach einem der vorangehenden Ansprüche,
wobei das 1,8-Cineol zur prophylaktischen und/oder therapeutischen Behandlung, insbesondere zur Suppression oder Abschwächung, eines im Rahmen einer COVID-19-Infektion auftretenden Hyperinflammationssyndroms eingesetzt wird, gegebenenfalls in Co-Medikation mit einem vorzugsweise systemisch applizierten Kortikosteroid, insbesondere Dexamethason; und/oder
wobei das 1,8-Cineol supprimierende oder abschwächende Wirkung in Bezug auf ein im Rahmen einer COVID-19-Infektion auftretendes Hyperinflammationssyndrom ausübt, gegebenenfalls in Co-Medikation mit einem vorzugsweise systemisch applizierten Kortikosteroid, insbesondere Dexamethason.

7. 1,8-Cineol zur Verwendung nach einem der vorangehenden Ansprüche,
wobei das 1,8-Cineol zur prophylaktischen und/oder therapeutischen Behandlung, insbesondere zur Suppression oder Abschwächung, einer im Rahmen einer COVID-19-Infektion auftretenden Hyperzytokinämie, insbesondere eines Zytokin-Freisetzungssyndroms, eingesetzt wird, gegebenenfalls in Co-Medikation mit einem vorzugsweise systemisch applizierten Kortikosteroid, insbesondere Dexamethason; und/oder
wobei das 1,8-Cineol eine supprimierende oder abschwächende Wirkung in Bezug auf eine im Rahmen einer COVID-19-Infektion auftretende Hyperzytokinämie, insbesondere in Bezug auf ein Zytokin-Freisetzungssyndrom, ausübt, gegebenenfalls in Co-Medikation mit einem vorzugsweise systemisch applizierten Kortikosteroid, insbesondere Dexamethason; und/oder
wobei das 1,8-Cineol zur Suppression oder Abschwächung der im Rahmen einer COVID-19-Infektion auftretenden Entzündungsmediation, insbesondere einer im Rahmen einer COVID-19-Infektion auftretenden Hyperzytokinämie, eingesetzt wird; und/oder
wobei das 1,8-Cineol eine supprimierende oder abschwächende Wirkung in Bezug auf eine im Rahmen einer COVID-19-Infektion auftretende Entzündungsmediation, insbesondere in Bezug auf eine im Rahmen einer COVID-19-Infektion auftretende Hyperzytokinämie, ausübt.

8. 1,8-Cineol zur Verwendung nach einem der vorangehenden Ansprüche,
wobei das 1,8-Cineol zur Suppression der Replikation von Corona-Viren, insbesondere zur Suppression der in Thrombocyten stattfindenden Replikation von Corona-Viren im Rahmen einer COVID-19-Infektion eingesetzt wird.

9. 1,8-Cineol zur Verwendung nach einem der vorangehenden Ansprüche,
wobei das 1,8-Cineol zur prophylaktischen und/oder therapeutischen Behandlung von bei COVID-19 auftretenden systemischen Organmitbeteiligungen, insbesondere bronchopulmonalen, kardialen und/oder renalen Organmitbeteiligungen, und/oder von bei COVID-19 auftretenden neurologischen Symptomen, Komplikationen oder Manifestationen eingesetzt wird.

10. 1,8-Cineol zur Verwendung nach einem der vorangehenden Ansprüche,
wobei das 1,8-Cineol als Induktor der NO-Produktion und/oder zur Behebung oder Linderung von bei COVID-19 auftretenden NO-Defizitzuständen im Körper eines erkrankten Patienten eingesetzt wird und/oder fungiert.

11. 1,8-Cineol zur Verwendung nach einem der vorangehenden Ansprüche,
wobei das 1,8-Cineol zur Behebung oder Linderung von bei COVID-19 auftretenden oxidativen und/oder inflammatorischen Zuständen im Körper eines erkrankten Patienten eingesetzt wird und/oder fungiert.

12. Arzneimittel oder Medikament, insbesondere Antivirusmittel, zur Verwendung bei der prophylaktischen und/oder therapeutischen Behandlung von COVID-19,
wobei das Arzneimittel oder Medikament, insbesondere Antivirusmittel, 1,8-Cineol zusammen mit mindestens einem pharmazeutisch verträglichen und/oder physiologisch unbedenklichen Exzipienten enthält,
wobei das 1,8-Cineol als magensaftresistente, aber dünndarmlösliche systemische Darreichungsform appliziert wird,
wobei das Arzneimittel oder Medikament 1,8-Cineol als Reinstoff mit einer Reinheit von mindestens 95 Gew.-%, bezogen auf das 1,8-Cineol, enthält und wobei das Arzneimittel oder Medikament das 1,8-Cineol frei von anderen Terpenen enthält.

13. Zusammensetzung, insbesondere pharmazeutische Zusammensetzung, zur Verwendung bei der prophylaktischen und/oder therapeutischen Behandlung von COVID-19,
wobei die Zusammensetzung 1,8-Cineol, insbesondere zusammen mit mindestens einem pharmazeutisch verträglichen und/oder physiologisch unbedenklichen Exzipienten, enthält,
wobei das 1,8-Cineol als magensaftresistente, aber dünndarmlösliche systemische Darreichungsform appliziert wird,
wobei die Zusammensetzung das 1,8-Cineol als Reinstoff mit einer Reinheit von mindestens 95 Gew.-%, bezogen auf das 1,8-Cineol, enthält und
wobei die Zusammensetzung das 1,8-Cineol frei von anderen Terpenen enthält.

14. 1,8-Cineol zur Verwendung nach einem der Ansprüche 1 bis 20, Arzneimittel oder Medikament zur Verwendung nach Anspruch 12 sowie Zusammensetzung zur Verwendung nach Anspruch 13,
wobei das 1,8-Cineol, insbesondere das Arzneimittel oder Medikament oder die Zusammensetzung, in pharmazeutisch wirksamen bzw. therapeutisch wirksamen Mengen verabreicht wird; und/oder
wobei das 1,8-Cineol, insbesondere das Arzneimittel oder Medikament oder die Zusammensetzung, zusammen mit mindestens einem weiteren Wirkstoff angewendet oder appliziert wird, insbesondere wobei der weitere Wirkstoff ausgewählt ist aus (i) entzündungshemmenden Wirkstoffen, insbesondere Kortikosteroiden, (ii) blutverdünnenden oder blutgerinnungshemmenden Wirkstoffen, (iiii) antiviral wirkenden Wirkstoffen, insbesondere synthetischen antiviralen Wirkstoffen, wie Remdesivir, oder proteinbasierten antiviralen Wirkstoffen, wie insbesondere monoklonalen Antikörpern, Immunglobulinen und Interferonen; (iv) kardiovaskulären Wirkstoffen, insbesondere Blutdrucksenkern und ACE-Hemmern; sowie deren Kombinationen.

15. Pharmazeutische Kombination zur Verwendung bei der prophylaktischen und/oder therapeutischen Behandlung von COVID-19,
wobei die pharmazeutische Kombination mindestens die nachfolgenden Komponenten (A) und (B) umfasst:
(A) 1,8-Cineol; sowie
(B) mindestens einen weiteren Wirkstoff, ausgewählt aus (i) entzündungshemmenden Wirkstoffen, insbesondere Kortikosteroiden, (ii) blutverdünnenden oder blutgerinnungshemmenden Wirkstoffen, (iiii) antiviral wirkenden Wirkstoffen, insbesondere synthetischen antiviralen Wirkstoffen, wie Remdesivir, oder proteinbasierten antiviralen Wirkstoffen, wie insbesondere monoklonalen Antikörpern, Immunglobulinen und Interferonen; (iv) kardiovaskulären Wirkstoffen, insbesondere Blutdrucksenkern und ACE-Hemmern; sowie deren Kombinationen;
wobei das 1,8-Cineol als magensaftresistente, aber dünndarmlösliche systemische Darreichungsform appliziert wird,
wobei das 1,8-Cineol als Reinstoff vorliegt, wobei das 1,8-Cineol mit einer Reinheit von mindestens 95 Gew.-%, bezogen auf das 1,8-Cineol, vorliegt und wobei das 1,8-Cineol frei von anderen Terpenen ist.

## Claims

1. 1,8-cineole for use in the prophylactic and/or therapeutic treatment of COVID-19,
wherein the 1,8-cineole is applied as an enteric-coated but small intestine-soluble systemic dosage form,
wherein the 1,8-cineole is present as a pure substance, wherein the 1,8-cineole is present with a purity of at least 95% by weight, based on the 1,8-cineole, and
wherein the 1,8-cineole is free from other terpenes.

2. 1,8-cineole for use according to claim 1,
wherein the 1,8-cineole is applied as a capsule, coated tablet, pill or tablet.

3. 1,8-cineole for use according to claim 1 or 2,
wherein the 1,8-cineole is administered at a daily dose in the range of from 1 to 5,000 mg/diem, in particular in the range of from 2 to 3,000 mg/diem, preferably in the range of from 5 to 2,500 mg/diem, preferably in the range of from 10 to 2,000 mg/diem, particularly preferably in the range of from 50 to 1,500 mg/diem.

4. 1,8-cineole for use according to any of the preceding claims,
wherein the 1,8-cineole is present and/or administered together with at least one physiologically acceptable excipient;
in particular wherein the physiologically acceptable excipient is miscible with and/or soluble in 1,8-cineole; and/or
in particular wherein the physiologically acceptable excipient is in the liquid or solid, preferably liquid, aggregation state at 20 °C and at atmospheric pressure; and/or
in particular wherein the physiologically acceptable excipient is selected from the group consisting of fatty acid esters, preferably triglycerides of fatty acids, particularly preferably medium-chain triglycerides, most preferably triglycerides of C₆-C₁₂ fatty acids.

5. 1,8-cineole for use according to any of the preceding claims,
wherein the 1,8-cineole is used or applied together with at least one other active substance;
in particular wherein the further active ingredient is selected from (i) anti-inflammatory active ingredients, in particular corticosteroids, (ii) blood-thinning or anticoagulant active ingredients, (iiii) antiviral active ingredients, in particular synthetic antiviral active ingredients, such as Remdesivir, or protein-based antiviral active ingredients, such as in particular monoclonal antibodies, immunoglobulins and interferons; (iv) cardiovascular agents, in particular antihypertensives and ACE inhibitors; and combinations thereof; and/or
in particular wherein the further active ingredient is used and/or administered separately, in particular spatially separated, from the 1,8-cineole but functionally related thereto, in particular in the form of a kit.

6. 1,8-cineole for use according to any of the preceding claims,
wherein the 1,8-cineole is used for the prophylactic and/or therapeutic treatment, in particular for the suppression or attenuation, of a hyperinflammation syndrome occurring in the context of a COVID-19 infection, optionally in co-medication with a preferably systemically applied corticosteroid, in particular dexamethasone; and/or
wherein the 1,8-cineole exerts a suppressive or attenuating effect in relation to a hyperinflammation syndrome occurring in the context of a COVID-19 infection, optionally in co-medication with a preferably systemically applied corticosteroid, in particular dexamethasone.

7. 1,8-cineole for use according to any of the preceding claims,
wherein the 1,8-cineole is used for the prophylactic and/or therapeutic treatment, in particular for the suppression or attenuation, of hypercytokinemia occurring in the context of a COVID-19 infection, in particular a cytokine release syndrome, optionally in co-medication with a preferably systemically applied corticosteroid, in particular dexamethasone; and/or
wherein the 1,8-cineole exerts a suppressive or attenuating effect in relation to hypercytokinemia occurring in the context of a COVID-19 infection, in particular in relation to a cytokine release syndrome, optionally in co-medication with a preferably systemically applied corticosteroid, in particular dexamethasone; and/or
wherein the 1,8-cineole is used to suppress or attenuate the inflammatory mediation occurring in the context of a COVID-19 infection, in particular hypercytokinemia occurring in the context of a COVID-19 infection; and/or
wherein the 1,8-cineole exerts a suppressive or attenuating effect in relation to inflammatory mediation occurring in the context of a COVID-19 infection, in particular in relation to hypercytokinemia occurring in the context of a COVID-19 infection.

8. 1,8-cineole for use according to any of the preceding claims,
wherein the 1,8-cineole is used to suppress the replication of corona viruses, in particular to suppress the replication of corona viruses taking place in thrombocytes in the context of a COVID-19 infection.

9. 1,8-cineole for use according to any of the preceding claims,
wherein the 1,8-cineole is used for the prophylactic and/or therapeutic treatment of systemic organ involvement occurring in COVID-19, in particular bronchopulmonary, cardiac and/or renal organ involvement, and/or of neurological symptoms, complications or manifestations occurring in COVID-19.

10. 1,8-cineole for use according to any of the preceding claims,
wherein the 1,8-cineole is used and/or functions as an inducer of NO production and/or to correct or alleviate NO deficiency states occurring in the body of a diseased patient with COVID-19.

11. 1,8-cineole for use according to any of the preceding claims,
wherein the 1,8-cineole is used and/or functions to correct or alleviate oxidative and/or inflammatory conditions in the body of a diseased patient associated with COVID-19.

12. Drug or medicament, in particular antiviral agents, for use in the prophylactic and/or therapeutic treatment of COVID-19,
wherein the drug or medicament, in particular antiviral agent, comprises 1,8-cineole together with at least one pharmaceutically acceptable and/or physiologically acceptable excipient,
wherein the 1,8-cineole is applied as an enteric-coated but small intestine-soluble systemic dosage form,
wherein the drug or medicament comprises 1,8-cineole as a pure substance with a purity of at least 95% by weight, based on the 1,8-cineole, and
wherein the drug or medicament comprises the 1,8-cineole free of other terpenes.

13. Composition, in particular pharmaceutical composition, for use in the prophylactic and/or therapeutic treatment of COVID-19,
wherein the composition comprises 1,8-cineole, in particular together with at least one pharmaceutically acceptable and/or physiologically acceptable excipient,
wherein the 1,8-cineole is applied as an enteric-coated but small intestine-soluble systemic dosage form,
wherein the composition comprises the 1,8-cineole as a pure substance with a purity of at least 95% by weight, based on the 1,8-cineole, and
wherein the composition comprises the 1,8-cineole free of other terpenes.

14. 1,8-cineole for use according to any one of claims 1 to 20, drug or medicament for use according to claim 12 and composition for use according to claim 13,
wherein the 1,8-cineole, in particular the drug or medicament or composition, is administered in pharmaceutically effective or therapeutically effective amounts; and/or
wherein the 1,8-cineole, in particular the drug or medicament or the composition, is used or applied together with at least one further active ingredient, in particular wherein the further active ingredient is selected from (i) anti-inflammatory active ingredients, in particular corticosteroids, (ii) blood-thinning or anticoagulant agents, (iiii) antiviral agents, in particular synthetic antiviral agents, such as Remdesivir, or protein-based antiviral agents, such as in particular monoclonal antibodies, immunoglobulins and interferons; (iv) cardiovascular agents, in particular antihypertensives and ACE inhibitors; and combinations thereof.

15. Pharmaceutical combination for use in the prophylactic and/or therapeutic treatment of COVID-19,
wherein the pharmaceutical combination comprises at least the following components (A) and (B):
(A) 1,8-cineole; and
(B) at least one further active ingredient selected from (i) anti-inflammatory active ingredients, in particular corticosteroids, (ii) blood-thinning or anticoagulant active ingredients, (iiii) antiviral active ingredients, in particular synthetic antiviral active ingredients, such as Remdesivir, or protein-based antiviral active ingredients, such as in particular monoclonal antibodies, immunoglobulins and interferons; (iv) cardiovascular agents, in particular antihypertensives and ACE inhibitors; and combinations thereof;
wherein the 1,8-cineole is applied as an enteric-coated but small intestine-soluble systemic dosage form,
wherein the 1,8-cineole is present as a pure substance, wherein the 1,8-cineole is present with a purity of at least 95% by weight, based on the 1,8-cineole, and
wherein the 1,8-cineole is free from other terpenes.

## Revendications

1. 1,8-cinéole pour une utilisation dans le traitement prophylactique et/ou thérapeutique du COVID-19,
le 1,8-cinéole étant appliqué sous forme d'administration systémique entérique (résistant au suc gastrique) mais soluble dans l'intestin grêle,
le 1,8-cinéole étant présent sous forme de substance pure, le 1,8-cinéole étant présent avec une pureté d'au moins 95 % en poids, par rapport au 1,8-cinéole, et
le 1,8-cinéole étant exempt d'autres terpènes.

2. 1,8-cinéole pour l'utilisation selon la revendication 1,
le 1,8-cinéole étant appliqué sous forme de capsule, de dragée, de pilule ou de comprimé.

3. 1,8-cinéole pour l'utilisation selon la revendication 1 ou 2,
le 1,8-cinéole étant administré à une dose journalière dans la plage de 1 à 5000 mg/diem, en particulier dans la plage de 2 à 3000 mg/diem, de préférence dans la plage de 5 à 2500 mg/diem, de préférence dans la plage de 10 à 2000 mg/diem, de manière particulièrement préférée dans la plage de 50 à 1500 mg/diem.

4. 1,8-cinéole pour l'utilisation selon l'une quelconque des revendications précédentes,
où le 1,8-cinéole est présent et/ou administré avec au moins un excipient physiologiquement acceptable;
en particulier où le excipient physiologiquement acceptable est miscible avec le 1,8-cinéole et/ou soluble dans celui-ci; et/ou
en particulier où le excipient physiologiquement acceptable est à l'état d'agrégat liquide ou solide, de préférence liquide, à 20°C et à la pression atmosphérique; et/ou
en particulier où le excipient physiologiquement acceptable est choisi dans le groupe des esters d'acides gras, de préférence des triglycérides d'acides gras, de manière particulièrement préférée des triglycérides à chaîne moyenne, de manière tout à fait préférée des triglycérides d'acides gras C₆-C₁₂.

5. 1,8-cinéole pour l'utilisation selon l'une quelconque des revendications précédentes,
le 1,8-cinéole étant utilisé ou appliqué conjointement avec au moins une autre substance active;
en particulier où l'autre substance active est choisie parmi (i) des substances actives anti-inflammatoires, en particulier des corticostéroïdes, (ii) des substances actives fluidifiant le sang ou anticoagulantes, (iiii) des substances actives antivirales, en particulier des substances actives antivirales synthétiques, comme le remdesivir, ou des substances actives antivirales à base de protéines, comme en particulier des anticorps monoclonaux, des immunoglobulines et des interférons; (iv) des agents cardiovasculaires, notamment des antihypertenseurs et des inhibiteurs de l'ECA; ainsi que leurs combinaisons; et/ou
en particulier où l'autre substance active est utilisée et/ou administrée séparément, en particulier spatialement séparée, du 1,8-cinéole mais fonctionnellement liée à celui-ci, en particulier sous la forme d'un kit.

6. 1,8-cinéole pour l'utilisation selon l'une quelconque des revendications précédentes,
le 1,8-cinéole étant utilisé pour le traitement prophylactique et/ou thérapeutique, en particulier pour la suppression ou l'atténuation, d'un -syndrome d'hyperinflammation apparaissant dans le cadre d'une infection par COVID-19, éventuellement en co-médication avec un corticostéroïde appliqué de préférence par voie systémique, en particulier la dexaméthasone; et/ou
le 1,8-cinéole exerçant une action supprimante ou atténuante vis-à-vis d'un syndrome d'hyperinflammation survenant dans le cadre d'une infection par COVID-19, éventuellement en co-médication avec un corticostéroïde appliqué de préférence par voie systémique, en particulier la dexaméthasone.

7. 1,8-cinéole pour l'utilisation selon l'une quelconque des revendications précédentes,
le 1,8-cinéole étant utilisé pour le traitement prophylactique et/ou thérapeutique, en particulier pour la suppression ou l'atténuation, d'une hypercytokinémie survenant dans le cadre d'une infection par COVID-19, en particulier d'un syndrome de libération de cytokines, éventuellement en co-médication avec un corticostéroïde appliqué de préférence par voie systémique, en particulier la dexaméthasone; et/ou
le 1,8-cinéole exerçant une action suppressive ou atténuante vis-à-vis d'une hypercytokinémie survenant dans le cadre d'une infection par COVID-19, en particulier vis-à-vis d'un syndrome de libération de cytokines, éventuellement en co-médication avec un corticostéroïde administré de préférence par voie systémique, en particulier la dexaméthasone; et/ou
le 1,8-cinéole étant utilisé pour supprimer ou atténuer la médiation inflammatoire se produisant dans le cadre d'une infection par COVID-19, en particulier une hypercytokinémie se produisant -dans le cadre d'une infection par COVID-19; et/ou
le 1,8-cinéole exerçant un effet supprimant ou atténuant par rapport à une -médiation inflammatoire se produisant dans le cadre d'une infection par COVID-19, en particulier par rapport à une hypercytokinémie se produisant dans le cadre d'une infection par COVID-19.

8. 1,8-cinéole pour l'utilisation selon l'une quelconque des revendications précédentes, le 1,8-cinéole étant utilisé pour la suppression de la réplication des virus Corona, en particulier pour la suppression de la réplication des virus Corona qui a lieu dans les thrombocytes dans le cadre d'une infection par COVID-19.

9. 1,8-cinéole pour l'utilisation selon l'une quelconque des revendications précédentes, le 1,8-cinéole étant utilisé pour le traitement prophylactique et/ou thérapeutique d'atteintes systémiques d'organes survenant lors de COVID-19, en particulier d'atteintes d'organes broncho-pulmonaires, cardiaques et/ou rénales, et/ou de symptômes, complications ou manifestations neurologiques survenant lors de COVID-19.

10. 1,8-cinéole pour l'utilisation selon l'une quelconque des revendications précédentes, le 1,8-cinéole étant utilisé et/ou agissant comme inducteur de la production de NO et/ou pour remédier ou atténuer les états de déficit en NO apparaissant dans COVID-19 dans le corps d'un patient malade.

11. 1,8-cinéole pour l'utilisation selon l'une quelconque des revendications précédentes,
le 1,8-cinéole étant utilisé et/ou agissant pour remédier ou atténuer les états oxydatifs et/ou inflammatoires apparaissant dans le cas de COVID-19 dans le corps d'un patient malade.

12. Médicament ou produit pharmaceutique, notamment agent antiviral, pour une utilisation dans le traitement prophylactique et/ou thérapeutique de COVID-19,
le médicament ou le produit pharmaceutique, en particulier l'agent antiviral, contenant du 1,8-cinéole conjointement avec au moins un excipient pharmaceutiquement acceptable et/ou physiologiquement acceptable,
le 1,8-cinéole étant appliqué sous forme d'administration systémique entérique (résistant au suc gastrique) mais soluble dans l'intestin grêle,
le médicament ou le produit pharmaceutique contenant du 1,8-cinéole sous forme de substance pure avec une pureté d'au moins 95 % en poids, par rapport au 1,8-cinéole, et le médicament ou le produit pharmaceutique contenant le 1,8-cinéole exempt d'autres terpènes.

13. Composition, notamment composition pharmaceutique, pour une utilisation dans le traitement prophylactique et/ou thérapeutique de COVID-19,
ladite composition contenant du 1,8-cinéole, en particulier conjointement avec au moins un excipient pharmaceutiquement acceptable et/ou physiologiquement acceptable,
le 1,8-cinéole étant appliqué sous forme d'administration systémique entérique (résistant au suc gastrique) mais soluble dans l'intestin grêle,
la composition contenant le 1,8-cinéole sous forme de substance pure avec une pureté d'au moins 95 % en poids, par rapport au 1,8-cinéole, et
ladite composition contenant le 1,8-cinéole exempt d'autres terpènes.

14. 1,8-cinéole pour l'utilisation selon l'une quelconque des revendications 1 à 20, médicament ou produit pharmaceutique pour l'utilisation selon la revendication 12 et composition pour l'utilisation selon la revendication 13,
où le 1,8-cinéole, en particulier le médicament ou la composition, est administré en quantités pharmaceutiquement efficaces ou thérapeutiquement efficaces; et/ou
où le 1,8-cinéole, en particulier le médicament ou la composition, est utilisé ou appliqué conjointement avec au moins un autre principe actif, en particulier où l'autre principe actif est choisi parmi (i) des principes actifs anti-inflammatoires, en particulier des corticostéroïdes, (ii) d'agents fluidifiant le sang ou anticoagulants, (iiii) d'agents antiviraux, en particulier d'agents antiviraux synthétiques, tels que le remdesivir, ou d'agents antiviraux à base de protéines, tels que notamment des anticorps monoclonaux, des immunoglobulines et des interférons; (iv) d'agents cardiovasculaires, notamment les antihypertenseurs et les inhibiteurs de l'ECA; ainsi que leurs combinaisons.

15. Combinaison pharmaceutique pour une utilisation dans le traitement prophylactique et/ou thérapeutique du COVID-19,
où la combinaison pharmaceutique comprend au moins les composants (A) et (B) suivants:
(A) 1,8-cinéole; ainsi que
(B) au moins une autre substance active choisie parmi (i) des substances actives anti-inflammatoires, en particulier des corticostéroïdes, (ii) des substances actives fluidifiant le sang ou anticoagulantes, (iiii) des substances actives antivirales, en particulier des substances actives antivirales synthétiques, comme le remdesivir, ou des substances actives antivirales à base de protéines, comme en particulier des anticorps monoclonaux, des immunoglobulines et des interférons; (iv) d'agents cardiovasculaires, notamment les antihypertenseurs et les inhibiteurs de l'ECA; ainsi que leurs combinaisons;
le 1,8-cinéole étant appliqué sous forme d'administration systémique entérique (résistant au suc gastrique) mais soluble dans l'intestin grêle,
le 1,8-cinéole étant présent sous forme de substance pure, le 1,8-cinéole étant présent avec une pureté d'au moins 95 % en poids, par rapport au 1,8-cinéole, et le 1,8-cinéole étant exempt d'autres terpènes.
